# EUROPÄISCHE PATENTSCHRIFT

(11) **EP 1 372 735 B1**
(45) Veröffentlichungstag und Bekanntmachung des Hinweises auf die Patenterteilung: **19.10.2011**
(21) Anmeldenummer: 02742858.0
(22) Anmeldetag: 15.03.2002
(51) Int. Cl.: A61K 47/48, C08B 31/10, C08B 31/18

(54) **HYDROXYALKYLSTÄRKE-WIRKSTOFF-KONJUGATE**
CONJUGATE OF HYDROXYALKYL STARCH AND AN ACTIVE AGENT
CONJUGUES D'AMIDON HYDROXYALKYLE ET D'AGENT ACTIF

(30) Priorität: 16.03.2001 DE 10112825
(43) Veröffentlichungstag der Anmeldung: 02.01.2004
(60) Teilanmeldung: 06002435.3
(73) Patentinhaber: Fresenius Kabi Deutschland GmbH, 61352 Bad Homburg v.d.H. (DE)
(72) Erfinder: SOMMERMEYER, Klaus, 61191 Rosbach (DE); EICHNER, Wolfram, 35510 Butzbach (DE); FRIE, Sven, 61389 Schmitten (DE); JUNGHEINRICH, Cornelius, 61350 Bad Homburg (DE); SCHARPF, Roland, 63691 Ranstadt (DE); LUTTERBECK, Katharina, 61169 Friedberg (DE); HEMBERGER, Jürgen, 63741 Aschaffenburg (DE); ORLANDO, Michele, 2585 DL Den Haag (NL)
(74) Vertreter: Altmann, Andreas
(86) Internationale Anmeldenummer: PCT/EP2002/002928
(87) Internationale Veröffentlichungsnummer: WO 2002/080979

(56) Entgegenhaltungen:
- EP-A- 0 304 183
- EP-A- 0 315 349
- WO-A-94/13697
- WO-A-98/01158
- WO-A-98/20905
- WO-A-99/49897
- DE-A- 3 029 307
- US-A- 5 217 998
- RICHTER A W ET AL: "ANTIBODIES AGAINST HYDROXYETHYLSTARCH PRODUCED IN RABBITS BY IMMUNIZATION WITH A PROTEIN-HYDROXYETHYLSTARCH CONJUGATE" INTERNATIONAL ARCHIVES OF ALLERGY AND APPLIED IMMUNOLOGY, BASEL, CH, Bd. 52, Nr. 1-4, 1976, Seiten 307-314, XP008014084 ISSN: 0020-5915
- MAOUT ET AL: "Hydroxyethyl starch conjugated to human hemoglobin for use in blood transfusion: Comparison with dextran conjugates" CARBOHYDRATES AND CARBOHYDRATE POLYMERS: ANALYSIS, BIOTECHNOLOGY, CONFORMATION, MODIFICATION, ANTIVIRAL AND OTHER COMMERCIAL APPLICATIONS, XX, XX, Bd. 1, 1993, Seiten 132-140, XP002107867
- SAKAI H ET AL: "Synthesis and physicochemical characterization of a series of hemoglobin-based oxygen carriers: Objective comparison between cellular and acellular types" BIOCONJUGATE CHEMISTRY, AMERICAN CHEMICAL SOCIETY, WASHINGTON, US, Bd. 11, Nr. 1, 2000, Seiten 56-64, XP002957844 ISSN: 1043-1802
- TOYAMA S ET AL: "Surface design of SPR-based immunosensor for the effective binding of antigen or antibody in the evanescent field using mixed polymer matrix" SENSORS AND ACTUATORS B, ELSEVIER SEQUOIA S.A., LAUSANNE, CH, Bd. 52, Nr. 1-2, 15. September 1998 (1998-09-15), Seiten 65-71, XP004157817 ISSN: 0925-4005
- CERNY ET AL: "A hydroxyethyl starch - hemoglobin polymer as a blood substitute" CLINICAL HEMORHEOLOGY, PERGAMON PRESS LTD, XX, Bd. 2, Nr. 4, 1982, Seiten 355-365, XP002107865
- SADRZADEH S M H ET AL: "THE LONG-ACTING PARENTERAL IRON CHELATOR, HYDROXYETHYL STARCH-DEFEROXAMINE, FAILS TO PROTECT AGAINST ALCOHOL-INDUCED LIVERINJURY IN RATS" JOURNAL OF PHARMACOLOGY AND EXPERIMENTAL THERAPEUTICS, AMERICAN SOCIETY FOR PHARMACOLOGY AND, US, Bd. 280, Nr. 2, 1. Februar 1997 (1997-02-01), Seiten 1038-1042, XP002065956 ISSN: 0022-3565
- LESNEFSKY E J ET AL: "HIGH-DOSE IRON-CHELATOR THERAPY DURING REPERFUSION WITH DEFEROXAMINE-HYDROXYETHYL STARCH CONJUGATE FAILS TO REDUCE CANINE INFARCT SIZE" JOURNAL OF CARDIOVASCULAR PHARMACOLOGY, NEW YORK, NY, US, Bd. 16, Nr. 4, 1. Oktober 1990 (1990-10-01), Seiten 523-528, XP002054497 ISSN: 0160-2446

## Beschreibung

Die vorliegende Erfindung betrifft Verbindungen, die ein Konjugat aus einer Hydroxyalkylstärke (HAS) und einem Wirkstoff umfassen, wobei die Hydroxyalkylstärke unmittelbar über eine Azomethingruppe -CH=N- oder eines Methylenamingruppe -CH₂-NH- Selektiv über die reduzierende Endgruppe der HAS kovalent an den Wirkstoff gebunden ist und wobei der wirhstoff ein protein, Oligo oder Polypeptid ist. Die Erfindung betrifft ferner Verfahren zur Herstellung eines kovalenten HAS-Wirkstoff-Konjugates, bei denen man HAS einen Wirkstoff in einem Reaktionsmedium selektiv über die reduzierende Endgruppe de HAS miteinander umsetzt, wobei die Umsetzung über die selektiv oxidierte reduzierende Endgruppe de HAS erfolgt oder wobei HAS und der Wirkstoff Unmittelbar unter Entstehung einer Schiff'schen Base miteinander umgesetzt werden dadurch gekennzeichnet, dass das Reaktionsmedium Wasser oder ein Gemisch von Wasser mit organischem Lösungsmittel, welches mindestens 10 Gew.-% Wasser umfaßt, ist und wobei der Wirkstoff ein Protein, Oligo oder Polypeptid ist. Die Erfindung betrifft auch die medizinische Verwendung der Konjugate.

### TECHNISCHER HINTERGRUND

Der klinische Einsatz vieler Pharma-Wirkstoffe wird durch eine Reihe von Problemen beeinträchtigt (vgl. Delgado et al., Critical Reviews in Therapeutic Drug Carrier Systems, Vol. 9 (3, 4), (1992) S. 249-304). Parenteral verabreichte native Proteine unterliegen beispielsweise der Ausscheidung durch das retikuloendotheliale System, die Leber, die Niere und die Milz. Die Ausscheidung erfolgt dabei in Abhängigkeit der Ladung der Kohlenhydratketten, der Präsenz zellulärer Rezeptoren für das Protein und von der Molekülform und -größe. Die Ausschlussgrenze der Glomerularfiltration der Niere liegt beispielsweise bei etwa 67 kD.

Als Folge proteolytischer Degradation ist ferner ein schneller Verlust der biologischen Aktivität zu beobachten.

Bakteriell exprimierte Proteine sowie andere rekombinante Proteine können eine erhöhte Immunogenität aufweisen und lebensbedrohliche Hypersensitivitätsreaktionen provozieren. Entsprechende Reaktionen verhindern natürlich die medizinische Verwendung dieser Produkte.

Aus diesem Grund wurde im Stand der Technik bereits seit Ende der 70er Jahre systematisch an der Verbesserung der Eigenschaften exogener Proteine durch chemische Modifikation, insbesondere Polymerisation oder Kopplung an makromolekulare Polymere geforscht. Viele Arbeiten konzentrierten sich dabei auf die Herstellung von Konjugaten aus Proteinen oder anderen Wirkstoffen einerseits und Polyethylenglykol (PEG) andererseits (vgl. US 4,179,337). Die Vorteile, die man sich von diesen Kopplungsreaktionen erhoffte, umfassen verbesserte in vivo Halbwertszeit der Proteine, verringerte Toxizität, verbesserte Stabilität und verbesserte Löslichkeit der Wirkstoffe (Abuchowski und Davis, Enzymes as drugs, Holcenberg und Rubberts, Herausgeber, S. 367-383, John Wiley & Sons N.Y. (1981).

Das Verfahren zur Kopplung der Wirkstoffe erwies sich jedoch als problematisch, da die aktive Gruppe des Proteins durch Kopplung an PEG inaktiviert wurde oder die Reaktionen das Reaktionsprodukt nicht in brauchbarer Ausbeute lieferten. Um eine spezifische Bindung zu erzielen, die die Aktivität des Wirkstoffs nicht beeinträchtigt, wurden aktive Gruppen in PEG oder den Wirkstoff eingeführt oder die Verbindungen mit einem Linker aneinander gebunden. Üblicherweise wird PEG dafür mit einer aktiven Gruppe versehen, welche nachfolgend kovalent an die kopplungsfähige Gruppe eines Proteins gebunden wird.

So wurde beispielsweise der Verlust der Bindungsaktivität von Antikörpern und deren Fragmenten nach deren Kopplung an PEG beschrieben (Kitamura et al., Cancer Res., Vol. 51 (1991), S. 4310-4315; und Pedley, et al., Br. J. Cancer, Vol. 79 (1994), S. 1126-1130). Zur Lösung dieses Problems schlagen Chapman et al. (Nature Biotech., Vol. 17 (1999), S. 780-783) vor, PEG an bestimmte Bindungsstellen des Antikörpers zu binden.

Der Aktivitätsverlust des Kopplungspartners wird ferner in WO 95/13090 beschrieben. Zur Lösung wird vorgeschlagen, PEG mit einer reaktiven Gruppe zu aktivieren und PEG über diese reaktive Gruppe in Gegenwart eines Tensides an α-Interferon zu binden. Als bevorzugte reaktive Gruppe wird N-Succinimid-Carbonat genannt, das unter den genannten Bedingungen mit der ε-Aminogruppe des Lysin eine Urethan-Bindung ausbilden soll.

Auch die WO 96/41813 offenbart Verfahren zur Herstellung eines Polymer-Polypeptid-Konjugates, bei denen das Polymer (insbesondere PEG) an einer spezifischen Stelle derivatisiert und anschließend an ein Polypeptid gebunden wird. Vorzugsweise wird dabei eine Amino-Oxi-Acetyl-Gruppe in PEG eingebracht und diese Verbindung anschließend an ein Polypeptid, insbesondere an IL-8, hG-CSF und IL-1 gebunden.

In der Literatur finden sich somit eine Vielzahl von Beispielen für entsprechende Konjugate; vgl. PEG-Insulin-Konjugate in US 4,179,337, bovine PEG-Hämoglobin-Konjugate in US 4,412,989, PEG-Ribonuklease-Konjugate und PEG-Superoxiddismutase-Konjugate in Veronese et al., Applied Biochem. Biotech., Vol. 11, 141-152 (1995), PEG-IL-2-Konjugate oder PEG-IFN-β-Konjugate in US 4,766,106, PEG-Polymyxin-Konjugate in WO 90/15628 und PEG-IL-2-Konjugate in WO 90/07939. Einige Konjugate befinden sich inzwischen in der klinischen Anwendung. Beispielsweise konnten die Eigenschaften des Enzyms Asparaginase durch Konjugatbildung mit PEG verbessert werden, und ein PEG-Asparaginase-Konjugat ist unter der Marke Oncaspar für die Krebstherapie kommerziell erhältlich. Kürzlich wurde ein an PEG gekoppeltes G-CSF von der US Food and Drug Administration zugelassen (Pegfilgastim). Eine große Anzahl weiterer pegylierter Produkte befindet sich in den verschiedenen Phasen der klinischen Entwicklung, darunter beispielsweise PEG-CDP870, PEG-Dronabinol, etc. (vgl. PEG-Pipeline unter www.enzon.com oder www.inhale.com).

Nicht nur Proteine sondern auch andere Verbindungen wurden nach diesem Schema an PEG und andere Polymere gekoppelt. WO 97/33552 und WO 97/38727 offenbaren beispielsweise die Kopplung von Paclitaxel an PEG und die Verwendung des Konjugates zur Behandlung von Tumoren. Die Verwendung eines PEG-Camptothecin-Konjugates zur Behandlung von Tumoren wird von der Firma Enzon in der klinischen Phase I untersucht.

Auch Antibiotika sind bereits an PEG gekoppelt worden. Dowling und Russell berichten beispielsweise über die Pharmakomkinetik eines Oxytetrazyklin-PEG-Konjugates (J. Vet. Pharmacol. Ther., Vol. 23 (2000), 107-110). Für den Erhalt neuer Funktionen wurden Antibiotika im Stand der Technik auch mittels anderer Verfahren derivatisiert. Beispielsweise wurde ein Depot-Penicillin hergestellt, welches ein Procain-Penicillin-Derivat ist, also das Salz des Penicillins mit der Procain-Base. Dieses Derivat besitzt eine verlängerte Wirkungsdauer und wird z.B. zur Therapie der Syphillis eingesetzt.

Kopplungsreaktionen mit mehr als zwei Verbindungen sind ebenfalls beschrieben worden. WO 93/23062 offenbart beispielsweise die Herstellung eines Kopplungsproduktes aus einem gegen ein B-Zell-Lymphom gerichteten Antikörper, aktiviertem PEG und einem Toxin.

PEG-Wirkstoff-Konjugate weisen jedoch keine natürliche Struktur auf, für die in vivo Abbauwege beschrieben wurden. Unter anderem aus diesem Grund sind neben den PEG-Konjugaten auch andere Konjugate und Protein-Polymerisate für die Lösung der oben genannten Probleme erzeugt worden. So gibt es eine Vielzahl von Verfahren zur Vernetzung verschiedener Proteine und Bindung von Proteinen an Makromoleküle (vgl. zusammenfassende Darstellung in Wong, S.S., "Chemistry of protein conjugation and cross linking", CRCS, Inc. (1993)).

Hydroxyethylstärke (HES) wird als Derivat eines natürlich vorkommenden Amylopektins im Körper durch α-Amylase abgebaut. Auch die Herstellung von HES-Protein-Konjugaten ist bereits im Stand der Technik beschrieben worden (vgl. HES-Hämoglobin-Konjugate in DE 26 16 086 oder DE 26 46 854).

Hämoglobin ist ein Protein, das als Blutersatz- und Sauerstoff-transport-Mittel (sog. "Hämoglobin-Based-Oxygen Carrier", HBOC) eine große klinische Bedeutung haben könnte. Obgleich der Bedarf an einem derartigen Produkt jedoch bereits frühzeitig erkannt wurde (vgl. Rabiner, J. Exp. Med. 126, (1967) 1127), hat bisher keines der bekannten HBOC-Produkte den Status eines zugelassenen Arzneimittels erreicht.

Das natürliche Hämoglobin besteht aus zwei α- und β-Peptidketten, die als prosthetische Gruppe jeweils ein Häm gebunden haben. Isolierte Hämoglobin-Moleküle sind jedoch sehr instabil und zerfallen rasch in die stabileren α,β-Dimere (MG 32 kDa). Die biologische Halbwertszeit von isoliertem Hämoglobin im Blutkreislauf liegt bei etwa 1 Stunde, da die Dimere schnell über die Nieren ausgeschieden werden. Dabei erzeugen die Dimere nephrotoxische Nebenwirkungen (vgl. Bunn & Jandl, J. Exp. Med. 129, (1967) 925-934). Entwicklungsarbeiten an derivatisierten Hämoglobin-Molekülen waren daher in erster Linie darauf gerichtet, Hämoglobin intramolekular zu vernetzen, zur Bildung von polymeren HBOC-Formen intermolekular zu verknüpfen und/oder an Polymere zu koppeln.

Die bekannten Hämoglobin-Konjugate werden beispielsweise in Xue und Wong (Meth. in Enzymol., 231 (1994), S. 308-322) und in DE 26 16 086 und DE 26 46 854 beschrieben. Letztere offenbart Verfahren mittels derer Hämoglobin an HES gebunden wird, indem HES zunächst mit Natriumperiodat umgesetzt wird. Dabei entstehen Dialdehyde, an die Hämoglobin gebunden wird. In ähnlicher Weise offenbaren die DE 30 29 307 A1 und die WO 99/49897 HES-Hämoglobin-Konjugate, bei denen die Hydroxylgruppen der HES zunächst durch Reaktion mit- Periodat in Aldehydgruppen überführt werden. Auch hier wird das Hämoglobin über die so erhaltenen Aldehydgruppen an die HES gebunden. Alternativ dazu offenbart die DE 30 29 307 A1 die Kupplung von Hämoglobin an HES, wobei die Hydroxylgruppen der HES zunächst durch Umsetzen mit einem Vernetzungsmittel, beispielsweise mit einem ein Alkylbromid enthaltenden Vernetzungsmittel, aktiviert werden, und das so erhaltene Zwischenprodukt mit dem Hämoglobin umgesetzt wird. In ähnliche Weise beschreibt die DE 26 16 086 die Kopplung von Hämoglobin an HES nach einem Verfahren, bei dem zunächst ein Vernetzungsmittel (z.B. Bromcyan) an HES gebunden wird und anschließend Hämoglobin an das Zwischenprodukt gebunden wird.

Ebenso offenbaren Richter et al. (International Archives of Allergy and Applied Immunology. 52, 307-315 (1976)) Konjugate zwischen HES und einem Protein, in diesem Fall BSA (bovine serum albumine), zu deren Herstellung OH-Gruppen der HES zunächst mit Bromcyan aktiviert werden und das erhaltene HES-Derivat anschließend mit dem Protein umgesetzt wird.

HES ist ein substituiertes Derivat des in Maisstärke zu 95 % vorkommenden Kohlenhydrat-Polymers Amylopektin. HES weist vorteilhafte rheologische Eigenschaften auf und wird zurzeit als Volumenersatzmittel und zur Hämodilutionstherapie klinisch eingesetzt (Sommermeyer et al., Krankenhauspharmazie, Vol. 8 (8), (1987), S.271-278; und Weidler et al., Arzneim.-Forschung/ Drug Res., 41. (1991) 494-498).

Amylopektin besteht aus Glucoseeinheiten, wobei in den Hauptketten α-1,4-glykosidische Bindungen vorliegen, an den Verzweigungg - stellen jedoch α-1,6-glykosidische Bindungen. Die physikalisch-chemischen Eigenschaften dieses Moleküls werden im Wesentlichen durch die Art der glykosidischen Bindungen bestimmt. Aufgrund der abgeknickten α-1,4-glykosidischen Bindung entstehen helikale Strukturen mit etwa 6 Glucose-Monomeren pro Windung.

Die physikalisch-chemischen als auch die biochemischen Eigenschaften des Polymers können durch Substitution verändert werden. Die Einführung einer Hydroxyethylgruppe kann durch alkalische Hydroxyethylierung erreicht werden. Durch die Reaktionsbedingungen kann die unterschiedliche Reaktivität der jeweiligen Hydroxylgruppe im unsubstituierten Glucosemonomer gegenüber der Hydroxyethylierung ausgenutzt werden, dadurch ist eine begrenzte Einflussnahme auf das Substitutionsmuster möglich.

Daher wird HES im Wesentlichen über die Molekulargewichtsverteilung und den Substitutionsgrad gekennzeichnet. Der Substitutionsgrad kann dabei als DS ("degree of substitution"), welcher auf den Anteil der substituierten Glucosemonomere aller Glucoseeinheiten Bezug nimmt, oder als MS ("molar substitution") beschrieben werden, womit die Anzahl von Hydroxyethylgruppen pro Glucoseeinheit bezeichnet wird.

HES-Lösungen liegen als polydisperse Zusammensetzungen vor, in denen sich die einzelnen Moleküle hinsichtlich des Polymerisationsgrades, der Anzahl und Anordnung der Verzweigungsstellen, sowie ihres Substitutionsmusters voneinander unterscheiden. HES ist somit ein Gemisch von Verbindungen mit unterschiedlichem Molekulargewicht. Dementsprechend wird eine bestimmte HES-Lösung durch ein durchschnittliches Molekulargewicht anhand statistischer Größen bestimmt. Dabei wird Mₙ als einfaches arithmetisches Mittel in Abhängigkeit von der Anzahl der Moleküle errechnet (Zahlenmittel), während M_{w}, das Gewichtsmittel, die masseabhängige Messgröße darstellt.

Eine selektive chemische Bindung von Proteinen an HES wurde bisher jedoch dadurch verhindert, dass die Aktivierung der HES nicht selektiv erfolgt. So resultieren die im Stand der Technik bekannten Protein-HES-Konjugate aus einer nicht-selektiven Kopplung von Bromcyan-aktivierter HES an Hämoglobin (vgl. DE 26 16 086). Entsprechende Verfahren können zu polydispersen Produkten mit uneinheitlichen Eigenschaften und potentiell toxischen Nebenwirkungen führen.

Von Hashimoto (Hashimoto et al., Kunststoffe, Kautschuk, Fasern, Vol, 9, (1992) S. 1271-1279) wurde erstmals ein Verfahren offenbart, in dem eine reduzierende Aldehyd-Endgruppe eines Saccharids selektiv oxidiert werden konnte, wobei ein reaktiver Ester (Lacton) gewonnen wurde.

Auf der Grundlage dieses Verfahrens offenbart WO 98/01158, dass Hämoglobin-Hydroxyethylstärke-Konjugate erhalten werden können, in denen Hämoglobin an HES selektiv über Amidbindungen zwischen freien Aminogruppen des Hämoglobins und der in oxidierter Form vorliegenden reduzierenden Endgruppe der HES miteinander verknüpft sind. Sowohl die in Hashimoto et al. offenbarten Verfahren als auch die Verfahren gemäß WO 98/01158 basieren jedoch auf einer Reaktion zwischen Saccharid (HES) und Protein (Hämoglobin) in organischem Lösungsmittel. Konkret wurde in dieser Veröffentlichung Dimethylsulfoxid (DMSO) verwendet.

Dem Fachmann ist jedoch bekannt, dass viele Proteine in organischen Lösungsmitteln eine Strukturänderung erleiden, welche sich in wässrigen Lösungen nicht zurückbildet. In der Regel geht mit der Strukturänderung auch ein Aktivitätsverlust einher. In jedem Fall ist das organische Lösungsmittel aufwendig zu entfernen, da für die geplante medizinische Verwendung bereits residuale Anteile organischer Lösungsmittel nicht akzeptabel sein können. Sogar die potentielle Gefahr von Verunreinigungen und Strukturänderungen der Proteine ist im Hinblick auf die geplante Verwendung auszuschliessen.

Der vorliegenden Erfindung liegt somit die Aufgabe zugrunde, verbesserte Hydroxyalkylstärke-Wirkstoff-Ronjugate und Verfahren zur deren Herstellung zur Verfügung zu stellen, die zu biologisch aktiven Konjugaten führen, welche im klinischen Alltag eingesetzt werden können. Eine weitere Aufgabe der vorliegenden Erfindung besteht darin, ein Verfahren zur Herstellung von Hydroxyalkylstärke-Wirkstoff-Konjugaten bereit zu stellen, bei denen nicht in nennenswertem Umfang Nebenprodukte erzeugt werden, da auch diese Nebenprodukte die anschließende Aufreinigung des Produktes in erheblichem Umfang beeinträchtigen.

Diese Aufgabe wurde nunmehr überraschendeweise durch ein Verfahren zur Herstellung eines kovalenten HAS-Wirkstoff Konjugates gelöst, bei dem man HAS und einen Wirkstoff selektiv über die reduzierende Endgruppe in einem Reaktionsmedium miteinander umsetzt, wobei die Umsetzung über die selektiv oxidierte reduzierende Endgruppe der HAS erfolgt oder wobei HAS und Wirkstoff unmittelbar unter Entstehung einer Schiffschen Base miteinander umgesetzt werden, dadurch gekennzeichnet, dass das Reaktionsmedium Wasser oder ein Gemisch von Wasser mit organischem Lösungsmittel, welches mindestens 10 Gew.% Wasser umfasst, ist, wobei der Wirkstoff ein Protein, Oligo- oder Polypeptid ist.

Die Erfindung betrifft ferner ein HAS-Wirkstoff Konjugat, bei dem der Wirkstoff unmittelbar an die HAS kovalent gebunden ist, erhältlich durch dieses Verfahren, wobei man eine Aminogruppe des Wirkstoffs mit HAS so umsetzt, dass eine Schiffsche Base gebildet wird.

Die Erfindung betrifft ferner ein HAS-Wirkstoff-Konjugat, bei dem der Wirkstoff kovalent über eine Azomethingruppe -CH=N- selektiv über die reduzierende Endgruppe der HAS unmittelbar an HAS gebunden ist, wobei der Wirkstoff ein Protein, Oligo- oder Polypeptid ist.

Die Erfindung betrifft ferner ein HAS-Wirkstoff-Konjugat, bei dem der Wirkstoff kovalent über eine Methylenamingruppe -CH₂-NH- selektiv über die reduzierende Endgruppe der HAS unmittelbar an HAS gebunden ist, wobei der Wirkstoff ein Protein, Oligo- oder Polypeptid ist

Die Erfindung betrifft ferner eine pharmazeutische Zusammensetzung, welche ein erfindungsgemäßes HAS-Wirkstoff Konjugat aufweist.

### KURZE BESCHREIBUNG DER FIGUREN:

- Fig. 1: GPC Chromatogramm der Kopplungsreaktion zwischen ox-HES 130 kD und HSA nach Verfahren A.III;
- Fig. 2: GPC Chromatogramm der Kopplungsreaktion zwischen ox-HES 130 kD und HSA nach Verfahren A.IV;
- Fig. 3: GPC Chromatogramm der Kopplungsreaktion zwischen ox-HES 130 kD und HSA nach Verfahren A.V und einer Reaktionszeit von 2 Stunden;
- Fig. 4: GPC Chromatogramm der Kopplungsreaktion zwischen ox-HES 130 kD und HSA, Verfahren A.V, Reaktionszeit über Nacht;
- Fig.5: GPC Chromatogramm der Kopplungsreaktion zwischen ox-HES 10 kD und HSA nach Verfahren A.V nach 2 Stunden (Fig. 5a) und über Nacht (Fig. 5b);
- Fig. 6: GPC Chromatogramm der Kopplungsreaktion zwischen ox-HES 130 kD und HSA nach Verfahren A.VII, nach 24 Stunden Reaktionszeit;
- Fig. 7: GPC Chromatogramm der Kopplungsreaktion zwischen ox-HES 130 kD und HSA nach Verfahren B.V;
- Fig. 8: SDS-PAGE und Western Blot verschiedener Kopplungsreaktionen zwischen HES und HSA;
- Fig. 9: SDS-PAGE und Western Blot verschiedener Kopplungsreaktionen zwischen HES und HSA;
- Fig. 10: Reaktionsschema für die Erstellung eines HES-DNA-Konjugates;
- Fig.11: Aufnahme eines Gels, das HES-DNA-Konjugate vor und nach einem Verdau mit einem Restriktionsenzym zeigt.

Im Rahmen der vorliegenden Erfindung wird eine chemische Verbindung als Wirkstoff bezeichnet, wenn die Verbindung geeignet ist, aktiver Bestandteil eines beliebigen Mittels für therapeutische oder diagnostische Zwecke zu sein. Vorzugsweise handelt es sich bei dem Wirkstoff um ein den aktiven Bestandteil eines Arzneimittels, also die Verbindung innerhalb einer Arzneimittelformulierung, welche nach Verabreichung an ein Subjekt eine physiolgische Wirkung erzielt.

Eine übersicht über die zugelassenen Arzneimittel und deren Wirkstoffe findet sich in der Roten Liste. Die in der Roten Liste genannten Wirkstoffe können für die Herstellung der HAS-Wirkstoff-Konjugate nach dem erfindungsgemäßen Verfahren verwendet werden. Gemäß der vorliegenden Erfindung umfaßt der Begriff Wirkstoff aber auch alle Verbindungen, deren Eignung für die diagnostische oder therapeutische Verwendung zwar bekannt ist, die jedoch aufgrund der oben dargestellten Probleme bisher für diesen Zweck nicht eingesetzt werden konnten. Vorzugsweise handelt es sich bei dem Wirkstoff um ein Vitamin, Impfstoff, Toxin, Antibiotikum (oder Antünfektivum), Antiarrhytmikum, Appetitzügler, Anästhetikum, Analgetikum, Antirheumatikum, Antiallergikum, Antiasthmatikum, Antidepressivum, Antidiabetikum, Antihistaminikum, Antihypertonikum, öder ein antineoplastisches Mittel. Strukturell handelt es sich um ein , Protein, Oligo- oder Polypeptid.

Die nach der vorliegenden Erfindung hergestellten Verbindungen behalten die Aktivität des Wirkstoffes und die vorteilhaften Eigenschaften der HAS. Als weitere Vorteile weisen die erfindungsgemäßen Konjugate weitere Vorteile, darunter eine verbesserte in vivo Halbwertszeit der Wirkstoffe, eine verringerte Toxizität, eine verbesserte Stabilität und/oder eine verbesserte Löslichkeit der Wirkstoffe auf.

Die HAS-Kette wird im Plasma nach der Verabreichung durch alpha-Amylase verkürzt. Die Aktivität des Kopplungsproduktes kann somit als Aktivität des nativen Kopplungsproduktes, also unmittelbar nach der Kopplung, oder als Aktivität des metabolisierten Kopplungsproduktes, also nach in vivo Metabolisierung des Kopplungsproduktes, bestimmt werden. Die in vivo Metabolisierung kann durch einen in vitro Abbau simuliert werden.

Die Aktivität des Wirkstoffes kann nach im Stand der Technik für diesen Stoff bekannten Verfahren bestimmt werden. Bei einem antineoplastischen Mittel wird die Aktivität beispielsweise als inhibitorische Konzentration (IC) oder bei einem antiinfektiven Mittel als minimale Hemmkonzentration (MHK) bestimmt. Die Bestimmung erfolgt vorzugsweise in vitro an geeigneten Zielzellen (vgl. Chow et al., Haematologica, Vol. 86 (2001), S. 485-493). Die in vitro Effekte können ferner durch ein relevantes Tiermodell bestätigt werden (vgl. beispielsweise das Mausmodell des Nierenzellkarzinoms, das in Changnon et al. beschrieben wurde, vgl. BJU Int., Vol. 88 (2001), S. 418-424).

Im Vergleich zu der ungekoppelten Substanz kann das native Kopplungsprodukt eine gesteigerte oder reduzierte Aktivität aufweisen. Die Aktivität wird jedoch vorzugsweise nicht mehr als 5fach, besonders bevorzugt nicht mehr als 3- oder 2-fach reduziert. Das metabolisierte Produkt weist vorzugsweise eine mit der ungekoppelten Substanz vergleichbare Aktivität auf, d.h. das metabolisierte Konjugat weist mindestens 50%, vorzugsweise mindestens 75% der Aktivität des Wirkstoffes vor der Kopplung auf, wobei ein Erhalt von mindestens 95% der Aktivität besonders bevorzugt ist.

Im Rahmen der vorliegenden Erfindung wird der Begriff "Hydroxyalkylstärke" dazu verwendet, um Stärkederivate zu bezeichnen, die mit einer Hydroxyalkylgruppe mit 1 bis 3 C-Atomen substituiert wurden. Die als "Hydroxyalkylstärke" bezeichnete Gruppe umfaßt somit Hydroxymethylstärke, Hydroxyethylstärke und Hydroxypropylstärke. Die Verwendung von Hydroxyethylstärke (HES) als Kopplungspartner ist für alle Ausführungsformen der Erfindung besonders bevorzugt.

Erfindungsgemäß ist es bevorzugt, dass die Hydroxyethylstärke ein mittleres Molekulargewicht (Gewichtsmittel) von 1-300 kDa, wobei ein mittleres Molekulargewicht von 5 bis 200 kDa besonders bevorzugt ist. Hydroxyethylstärke kann ferner einen molaren Substitutionsgrad von 0,1-0,8 und ein Verhältnis von C₂:C₆-Substitution im Bereich von 2-20, jeweils bezogen auf die Hydroxyethylgruppen, aufweisen.

Für die Kopplung des Wirkstoffes an die HAS kann es in einem ersten Schritt notwendig sein, eine aktive Gruppe in den Wirkstoff und/oder in die HAS einzuführen. Entsprechende aktive Gruppe können beispielsweise Thiol- oder Aminogruppen sein (vgl. Beispiele).

Ferner können Wirkstoff und HAS unter Verwendung eines Linkers aneinander gekoppelt werden. Als Linker kann ein beliebiges Vernetzungsmittel eingesetzt werden. Zahlreiche Vernetzungsmittel, wie beispielsweise SMCC (Succinimidyl-4-(N-maleimidomethyl)cyclohexan-1-carboxylat; vgl. Beispiel 7), sind kommeriziell erhältlich und dem Fachmann geläufig (vgl. alphabetische Liste der "Cross-linking Reagents" im Produktkatalog der Firma Perbio und www.piercenet.com).

Gemäß einer bevorzugten Ausführungsform betrifft die vorliegende Erfindung Verbindungen, die ein Konjugat aus HAS und einem antineoplastischen Wirkstoff umfassen und deren Verwendung zur Behandlung von Tumoren.

Erfindungsgemäß wurde nunmehr überraschenderweise festgestellt, dass HAS-Wirkstoff-Konjugate, welche einen antineoplastischen Wirkstoff umfassen, eine verbesserte toxische Wirkung gegenüber Tumorzellen und/oder eine verringerte Toxizität für andere Zellen aufweisen. Die Konjugate ermöglichen damit eine größere therapeutische Breite.

Die Plasma-Halbwertszeit der Konjugate wird signifikant verlängert. Dies ermöglicht ein Durchbrechen der Reparaturmechanismen in Tumor-Zellen durch längere Exposition. Gleichzeitig ermöglicht die vorliegende Erfindung ein langsameres Anfluten, insbesondere in gesundem Gewebe, wodurch eine geringere Peak-Konzentration und eine verbesserte Verträglichkeit für den Patienten erreicht wird.

Für die Herstellung der erfindungsgemäßen Konjugate wird als antineoplastischer Wirkstoff Verwendet.

Der antineoplastische Wirkstoff wird erfindungsgemäß selektiv über die reduzierende Endgruppe der HAS an HAS gekoppelt wobei die Umsetzung über die selektiv oxidierte reduzierende Endgruppe der HAS erfolgt oder wobei HAS und der Wirkstoff unmittelbar unter Entstehung einer Schiffs'chen Base miteinander umgesetzt werden.

Gemäß einer weiteren Ausführungsform betrifft die vorliegende Erfindung Verfahren zur Herstellung einer Verbindung, die ein Konjugat aus HAS und einem antineoplastischen Wirkstoff umfaßt. Das Verfahren umfaßt Schritte, bei denen man HAS selektiv über die reduzierende Endgruppe der HAS kovalent an einen antineoplastischen Wirkstoff koppelt und das Konjugat isoliert wobei die Umsetzung über die selektiv oxidierte reduzierende Endgruppe der HAS erfolgt oder wobei HAS und der Wirkstoff unmittelbar unter Entstehung einer Schiffs'chen Base miteinander umgesetzt werden.

Ferner betrifft die Erfindung pharmazeutische Zusammensetzungen, welche eine Verbindung umfassen, die ein Konjugat aus HAS und einem antineoplastischen Wirkstoff aufweisen. Die pharmazeutische Zusammensetzung kann ferner einen pharmazeutisch verträglichen Träger und/oder ein Zytokin umfassen. Das Zytokin ist vorzugsweise IL-2, α-Interferon, γ-Interferon.

Die pharmazeutische Zusammensetzung kann in einer beliebigen im Stand der Technik bekannten Applikationsform vorliegen. Beispielsweise kann die Zusammensetzung unter Umständen zur oralen oder parenteralen Verabreichung formuliert sein. Die Formulierung der Zusammensetzung erfolgt nach im Stand der Technik üblichen Verfahren. Neben dem Wirkstoff enthält die Zusammensetzung üblicherweise einen pharmazeutisch verträglichen Träger und einen oder mehrere Hilfsstoffe; sowie gegebenenfalls Konservierungsmittel, Lösungsvermittler, etc.

Schließlich betrifft die vorliegende Erfindung die Verwendung einer Verbindung, die ein Konjugat aus HAS und einem antineoplastischen Wirkstoff umfaßt wobei de Wirkstoff unmiltelbar über eine Azomethingruppe -CH=N- oder eine Methylenamingruppe-CH₂-NH-selektiv über die reduzierende Endgruppe de HAS an HAS gekuppelt ist, zur Herstellung eines Arzneimittels zur Behandlung von Tumoren und/oder deren Metastasen, insbesondere zur Behandlung von urologischen Tumoren und/oder Metastasen urologischer Tumore, zur Behandlung von Metastasen des Nierenzellkarzinoms, oder zur Behandlung lymphatischer Systemerkrankungen, wie beispielsweise CLL, Hodgkin-Lymphom, NHL, multiples Myelom, Morbus Waldenström. Auch gemäß dieser Ausführungsform der Erfindung kann das Arzneimittel ferner ein Zytokin, beispielsweise IL-2, α-Interferon, γ-Interferon, umfassen.

Die Verwendung der erfindungsgemäßen Verbindungen zur Herstellung eines Arzneimittels zur Behandlung von urologischen Tumoren und/-oder Metastasen urologischer Tumore, beispielsweise zur Behandlung von Metastasen des Nierenzellkarzinoms ist besonders bevorzugt. Eine kurative Therapie des Nierenzellkarzinoms ist zur Zeit weder mit einer Kombinations-Chemotherapie noch mit Mitomycin C alleine zu erreichen. Dies kann an der ungünstigen Pharmakokinetik der Verbindung liegen, da der Anteil der renalen Elimination nur bei ca. 18% liegt. Da HAS nahezu vollständig über die Nieren eliminiert wird, weist das Konjugat einen höheren Prozentsatz der renalen Elimination im Vergleich zu der nichtkonjugierten Substanz auf. Diese Ausführungsform der vorliegenden Erfindung nutzt die intrazelluläre Zwischenspeicherung der HAS. Insbesondere hoch-substituierte HAS-Sorten (HAS 200/0,62) weisen eine verstärkte intrazelluläre Speicherung, im Extremfall sogar eine Überladung auf. Dieses Phänomen ist auch im Bereich des proximalen Tubulus beobachtet worden (Peron et al., Clinical Nephrology, Vol. 55 (2001), S.408-411).

Gemäß dieser Ausführungsform stellt die vorliegende Erfindung somit eine Anreicherung eines antineoplastischen Wirkstoffs in bestimmten Zielzellen oder -geweben zur Verfügung. Die verbesserte Pharmakokinetik der Konjugate ermöglicht es somit bei niedrigeren systemischen Konzentrationen eine wesentlich höhere Konzentration in den Zellen des Zielorgans zu erreichen. Diese medizinische Verwendung wird bevorzugt bei dem klarzelligen und dem chromophilen Nierenkarzinom einggsetzt, welche zusammen etwa 90% aller histologischer Typen ausmachen.

Gemäß einer alternativen Ausführungsform betrifft die Erfindung die Verwendung der erfindungsgemäßen Verbindungen zur Herstellung eines Arzneimittels zur Behandlung lymphatischer Systemerkrankungen, beispielsweise CLL, Hodgkin-Lymphom, NHL, multiples Myelom, Morbus Waldenström. Durch die erfindungsgemäße Kopplung von HAS an einen antineoplastischen Wirkstoff wird die intrazelluläre Aufnahme der Wirkstoffe in Abhängigkeit der Kettenlänge und des Sustitutionsgrads verlangsamt. Ferner haben radioaktive kinetische Untersuchungen gezeigt, dass HAS in bestimmten Organen, darunter lymphatische Organe, länger als im Gesamtkörper gespeichert wird (vgl. Bepperling et al., Crit. Care, Vol.3, Suppl. 1 (1999), S.153). Es erfolgt somit eine Anreicherung der Konjugate in den Zielzellen, was eine verbesserte Pharmakokinetik bei geringerer systemischer Toxizität zur Folge hat.

Die Erfindung betrifft ferner die Verwendung der erfindungsgemäßen Verbindungen zur Herstellung eines Arzneimittels zur Behandlung von kutanen/lokalen primären Malignomen oder deren Metastasen. Dabei werden zwei Effekte genützt, die gezielte, gesteigerte Aufnahme durch die genannten Gewebe und der verzögerte Abtranspo.rt des HAS-Konjugates aus dem Gewebe. Beides führt zu einer Anreicherung des Konjugates in den Zielzellen.

Die Erfindung betrifft ferner die Verwendung der erfindungsgemäßen Verbindungen zur Herstellung eines Arzneimittels zur Behandlung von hämatologischen Systemerkrankungen oder onkologischen Erkrankungen, beispielsweise von nicht-kleinzelligen und kleinzelligen Bronchialkarzinomen, Mammakarzinomen, Ösophagus-Plattenepithelkarzinomen, Nierenzellkarzinomen, Hodenkarzinomen, maligne Melanome, ALL oder CML. Insbesondere bei der Verwendung der Konjugate zur Behandlung des Nierenzellkarzinoms ergeben sich Vorteile aufgrund der starken Anreicherung der Verbindung in dem betroffenen Gewebe durch die größere Hydrophilie des Konjugates und der daraus folgenden stärkeren renalen Elimination. Für diese Ausführungsform der Erfindung ist die Verwendung von Vindesin als Wirkstoff besonders bevorzugt.

Die Erfindung betrifft ferner die Verwendung der erfindungsgemäßen Verbindungen zur Herstellung eines Arzneimittels, wobei die Verbindung als Kombinationstherapie mit einem oder mehreren weiteren antineoplastischen Wirkstoffen oder Zytokinen eingesetzt wird. Die Kombinationstherapie kann durch Verabreichung eines Mittels erfolgen, in dem alle Wirkstoffe vorliegen, oder durch Verabreichung von zwei verschiedenen oder mehr Mitteln, in denen jeweils ein Wirkstoff formuliert wurde.

Die vorliegende Erfindung stellt ferner Verfahren zur Herstellung eines Arzneimittels zur Verfügung, das ein Zytokin und eine erfindungsgemäße Verbindung enthält und für neue Kombinationstherapien geeignet ist. Entsprechende Mittel sind insbesondere für die Behandlung des fortgeschrittenen Nierenzellkarzinoms geeignet.

Die vorliegende Erfindung betrifft Verbindungen, die ein Konjugat aus HAS und einem antiinfektivem Wirkstoff, beziehungsweise einem Antibiotikum umfassen, sowie deren Verwendung zur Behandlung von Infektionserkrankungen.

Das Eindringen von Mikroorganismen (Viren, Bakterien, Pilze, Protozoen) in einen Makroorganismus (Pflanze, Tier, Mensch) und die Vermehrung in diesem wird als Infektion bezeichnet. Entstehung und Verlauf einer Infektionskrankheit hängen im wesentlichen von der Pathogenität des Mikroorganismus und von der Immunität des Makroorganismus ab.

Zur Bekämpfung von Infektionskrankheiten werden seit Jahrzehenten antiinfektive wirkstoffe als Chemotherapeutika eingesetzt.

Bereits 1928 wurde Penicillin anhand der Eigenschaft des Wirkstoffs, Staphylokokken-freie Zonen auf Kulturschalen zu bilden, von A. Flemings identifiziert. Penicillin war auch das erste Antibiotikum, das im industriellen Maßstab gewonnen werden konnte und große Bedeutung in der klinischen Praxis erlangte.

Als Penicillin werden heute Wirkstoffe aus einer Gruppe von β-Laktam-Antibiotika bezeichnet, die von einem Pilz der Art Penicilium (z.B. P. chrysogenurn und P. notatum) gebildet werden. Die bakteriozide Wirkung beruht auf der Blockierung der Synthese der Bakterienzellwand. Das Penicillin inaktiviert das bakterielle Enzym Transpeptidase, wodurch die Quervernetzung der Polysaccharidketten des Mureins der Zellwand verhindert wird.

Seit der Entdeckung wurden zahllose Wirkstoffe isoliert und synthetisiert, die das Wachstum von Mikroorganismen hemmen oder diese abtöten. Die meisten Antibiotika stammen von Streptomyceston, die in ihrer möglichen Bedeutung für die Pathogenese der Arteriosklerose erst kürzlich erkannt wurde (Stille und Dittmann, Herz, Vol.23 (1998), S.185-192) können intrazelluläre, mit Depotwirking versehene Antibiotika einen wesentlichen Fortschritt in Therapie und Prophylaxe bedeuten.

Erfindungsgemäß wurde nunmehr überraschenderweise festgestellt, dass die Kopplung antiinfektiver Wirkstoffe an HAS zu einer Verbesserung der pharmakokinetischen Eigenschaften der Wirkstoffe, insbesondere zu einer verlängerten in vivo Halbwertszeit, einer verbesserten intrazellulären Aufnahme und/oder Wirksamkeit des Wirkstoffs führt.

Erfindungsgemäß wird als antiinfektiver Wirkstoff ein Glycopeptid-Antibiotika verwendet.

Dabei kann es sich beispielsweise um Vancomycin handeln.

Die Kopplung zwischen dem antiinfektiven Wirkstoff und der Hydroxyethylstärke erfolgt über die reduzierenden Endgruppen der Hydroxyethylstärke.

gemäß einer weiteren Ausführungsform der vorliegenden Erfindung ist der antiinfektive Wirkstoff über einen Linker an die Hydroxyethylstärke gekoppelt.

Die vorliegende Erfindung umfaßt ferner pharmazeutische Zusammensetzungen, welche eine erfindungsgemäße Verbindung aufweisen. Üblicherweise enthalten die pharmazeutischen Zusammensetzungen ferner einen pharmazeutisch verträglichen Träger.

Demgemäß werden Verfahren zur Erstellung eines kovalenten HAS-Wirkstoff-Konjugates zur Verfügung gestellt, bei denen man HAS und mindestens einen Wirkstoff in einem Reaktionsmedium miteinandere umsetzt. Das Reaktionsmedium ist dadurch gekennzeichnet, dass es Wasser oder ein Gemisch von Wasser mit organischem Lösungsmittel, welches mindestens 10 Gew.-% Wasser umfaßt, ist.

Das Reaktionsmedium des erfindungsgemäßen Verfahren umfaßt mindestens 10 Gew.-%, bevorzugt mindestens 50 Gew.-%, insbesondere mindestens 80 Gew.-%, wie beispielsweise .90 Gew.-%, oder sogar bis zu . 100 ges.-%, Wasser, und dementsprechend höchstens 90% Gew.-%, bevorzugt höchstens 50% Gew.-%, insbesondere höchstens 20 Gew.-%, beispielsweise 10 Gew.-%, oder sogar bis zu 0 Gew.-%, organisches Lösungsmittel Die Reaktion findet also in einer wäßrigen Phase statt. Das bevorzugte Reaktionsmedium ist Wasser .

Das erfindungsgemäße Verfahren ist schon deshalb von Vorteil, weil nicht zwangsläufig toxikologisch bedenkliches Losungsmittel eingesetzt werden muss und demzufolge bei dem erfindungsgemäß hergestellten Produkt die Entfernung, auch geringer Reste toxikologisch bedenklicher Lösungsmittel entfällt, wie sie nach dem bekannten Verfahren immer zwingend ist, um die unerwünschte Verunreinigung mit Lösungsmittel zu vermeiden. Weiterhin kann die nach dem bisherigen Verfahren notwendige zusätzliche Qualitätskontrolle auf Reste toxikologisch bedenklicher Lösungsmittel unterbleiben, weil das erfindungsgemäße Verfahren die Verwendung von toxikologisch nicht bedenklichen Lösungsmitteln bevorzugt. Erfindungsgemäss bevorzugte Lösungmittel sind beispielsweise toxikologisch unbedenkliche protische Lösungsmittel wie Ethanol oder Propylenglykol.

Weiterhin ist ein Vorteil des erfindungsgemässen Verfahrens, dass durch organische Lösungsmittel induzierte irreversible oder reversible Strukturänderungen von Proteinen oder Peptiden grundsätzlich vermieden werden, die bei Verfahren in organischen Lösungsmitteln nicht systematisch ausgeschlossen werden können. Die nach den erfindungsgemässen Verfahren erhaltenen Polypeptide können demzufolge von den in DMSO hergestellten verschieden sein.

Erfindungsgemäß wurde ferner überraschenderweise festgestellt, dass eine Kopplung von HAS an Wirkstoffe in einer wässrigen Lösung vorgenommen werden kann, ohne dass dabei in nennenswertem Umfang Nebenreaktionen zu beobachten sind. Das erfindungsgemäße Verfahren führt somit unmittelbar zu verbesserten Produkten in großer Reinheit. Das erfindungsgemäße Verfahren ermöglicht somit erstmals die einfache Herstellung von HAS-Wirkstoff-Konjugaten, in denen der Wirkstoff in aktiver Form vorliegt und die vorteilhaften Eigenschaften der HAS erhalten bleiben. Zur Isolierung des HAS-Wirkstoff-Konjugates aus dem Reaktionsgemisch sind keine besonderen Verfahren erforderlich, da die Reaktion in der wässrigen Phase erfolgt, also nicht zwangsläufig organische Lösungsmittel abgereinigt werden müssen.

Erfindungsgemäß ist es bevorzugt, dass HAS unmittelbar an eine ε-NH₂-Gruppe, α-NH₂-Gruppe, SH-Gruppe, COOH-Gruppe oder -C(NH₂)₂-Gruppe des Wirkstoffes bindet. Alternativ dazu kann eine weitere reaktive Gruppe in HAS eingeführt werden oder die Bindung zwischen HAS und dem Wirkstoff über einen Linker erfolgen. Die Verwendung von entsprechenden Linkern zur Bindung von Wirkstoffen an PEG ist im Stand der Technik bekannt. Die Verwendung von Aminosäuren, insbesondere Glycin, Alanin, Leucin, Isoleucin, und Phenylalanin, sowie von Hydrazin- und Oxylamin-Derivaten als Linker, wie in WO 97/38727 und EP 605 963 offenbart, ist bevorzugt.

Gemäß einer Ausführungsform des Verfahrens der vorliegenden Erfindung wird HAS vor Bindung an den Wirkstoff oxidiert, wobei eine selektive Oxidation der reduzierenden Endgruppe der HAS durchgeführt wird. Dieses Vorgehen ermöglicht Verfahren, bei denen die oxidierte reduzierende Endgruppe der HAS mit einer Aminogruppe des Wirkstoffes unter Ausbildung eines Amids reagiert. Diese Ausführungsform weist den besonderen Vorteil auf, dass eine spezifische Bindung zwischen HAS und den Wirkstoffen und somit ein besonders homogenes Produkt erzielt wird.

Dabei läßt man HAS mit oxidierter reduzierender Endgruppe und den Wirkstoff vorzugsweise mindestens 12, besonders bevorzugt mindestens 24 Stunden miteinander reagieren. Ferner kann es wünschenswert sein, einen beliebigen Aktivator, beispielsweise Ethyldimethyl-aminopropyl-Carbodümid (EDC), zuzugeben. Das Mol-Verhältnis zwischen HAS und Wirkstoff während der Reaktion kann beliebig gewählt werden, liegt üblicherweise im Bereich von HAS: Wirkstoff von 20:1 bis 1:20, wobei ein Verhältnis von 6:1 bis 1:6 besonders bevorzugt ist. Die besten Ergebnisse wurden bei einem Mol-Verhältnis von HAS:Wirkstoff von etwa 2:1 erzielt.

Gemäß einer alternativen Austührungsform der volugenden erfindung werden HAS und der Wirkstoff unmittelbar miteinander umgesetzt wobei eine Schiff'sche Base zwischen HAS und Wirkstoff als Zwischenprodukt entsteht. Die Azomethingruppe -CH=N- der Schiff'schen Base kann anschließend unter formaler Addition von <2H> zu einer Methylenamingruppe -CH₂-NH- reduziert werden. Für diese Reduktion kann der Fachmann eine Vielzahl von im Stand der Technik bekannten Reduktionsmitteln verwenden, wobei die Reduktion mittels BH, besonders bevorzugt ist.

Die Kopplung des HAS kann an eine beliebige Gruppe des Wirkstoffes erfolgen. Die Kopplung wird vorzugsweise so vorgenommen, dass das metabolisierte Konjugat mindestens 50%, vorzugsweise mindestens 75% der Aktivität des Wirkstoffes vor der Kopplung aufweist, wobei ein Erhalt von mindestens 95% der Aktivität besonders bevorzugt ist. Die Kopplungsreaktion kann natürlich auch so gesteuert werden, dass die Bindung des HAS ausschließlich an eine oder meherere bestimmte Gruppen des Wirkstoffes erfolgt, beispielsweise an Lysin- oder oder Cystein-Reste eines Peptids. Besondere Vorteile ergeben sich, wenn man HAS über die oxidierte reduzierende Endgruppe an eine oder mehrere bestimmte Gruppen des wirkstoffes bindet, da bei einem entsprechenden Vorgehen homogene HAS-Wirkstoff-Konjugate erhalten werden.

Gemäß des Verfahrens der vorliegenden Erfindung werden als Ausgangsstoffe für die Reaktion HAS und ein Protein oder ein Peptid eingesetzt. Dabei können beliebige therapeutische oder diagnostische Proteine natürlichen oder rekombinanten Ursprungs verwendet werden. Eine Liste der derzeit auf dem Markt befindlichen Wirkstoffe rekombinanter Herstellung findet sich in Pharma Business, July/August 2000, Seiten 45 bis 60. Die vorliegende Erfindung umfaßt die Herstellung von HAS-Wirkstoff-Konjugaten, die irgend einen der in dieser Veröffentlichung genannten Wirkstoffe umfassen.

Die Herstellung von Konjugaten unter Verwendung von Zytokinen, beispielsweise Interferonen, Interleukinen, Wachstumsfaktoren, Enzymen, Enzym-Inhibitoren, Rezeptoren, Rezeptor-Fragmenten, Insulin, Faktor VIII, Faktor IX, Antibiotika (oder Antiinfektiva), Peptid-Antibiotika, viralen Hüll-Proteinen, Hämoglobinen, Erythropoietin, Albuminen, hTPA, Antikörpern, Antikörperfragmenten, Einzelketten-Antikörpern, oder ein Derivat derselben ist besonders bevorzugt. Besondere Vorteile ergeben sich bei der Verwendung von rekombinanten Proteinen oder Peptiden in dem erfindungsgemäßen Verfahren. Wie bereits ausgeführt, können entsprechende Proteine häufig aufgrund ihrer für Menschen antigenen Eigenschaften nicht als Wirkstoffe eingesetzt werden. Nach Kopplung an HAS durch die erfindungsgemäßen Verfahren verringert sich jedoch die Immunogenität der rekombinanten Proteine, was den medizinische Einsatz am Menschen ermöglicht.

Besondere Vorteile ergeben sich ferner bei der Kopplung von HAS an kurzkettige Proteine und kleinere Peptide. Derzeit werden eine Vielzahl von Peptidbanken erstellt, beispielsweise Phagen-Display-Banken, bei denen kurze Oligopeptide (beispielsweise 3-bis 20-mere) auf der Oberfläche von Phagen exprimiert werden. Ferner werden Antikörper aus einer einzigen Polypeptidkette (sogenannte "single chain antibodies", Einzelketten-Antikörper) in Bakterien oder auf der Oberfläche von Phagen exprimiert. Diese Banken werden auf bestimmte Wirkstoff- oder Bindungs-Aktivität hin durchgesehen. Die therapeutische und diagnostische Verwendung entsprechender Peptid-Wirkstoffe oder Antikörper scheitert jedoch bislang daran, dass diese im Organismus aufgrund ihrer geringen Größe sehr schnell ausgeschieden werden (vgl. Chapman et al., 1999, a.a.O.). Nach dem erfindungsgemäßen Verfahren können diese Peptide vorteilhaft an HAS gekoppelt werden, und weisen eine in vivo Halbwertszeit auf, die eine Verwendung als Wirkstoff ermöglicht.

Erfindungsgemäß kann beliebige physiologisch verträgliche HES als Ausgangsmaterial verwendet werden. HES mit einem mittleren Molekulargewicht (Gewichtsmittel) von 1 bis 300 kDa, insbesondere von 1 bis 150 kDa ist bevorzugt. HES mit einem mittleren Molekulargewicht von 2 bis 40 kD ist besonders bevorzugt. HES weist vorzugsweise einen molaren Substitutionsgrad von 0,1 bis 0,8 und ein Verhältnis von C_{2 :} C₆-Substitution im Bereich von 2 bis 20, jeweils bezogen auf die Hydroxyethylgruppen, auf.

Die Erfindung betrifft ferner die nach den obigen Verfahren erhältlichen HAS-Wirkstoff-Konjugate. Diese Konjugate weisen vorteilhafte Eigenschaften auf, nämlich hohe Aktivität des Wirkstoffs, geringe Immunogenität, lange Verweilzeit im Körper und exzellente rheologische Eigenschaften, welche den medizinischen Nutzen der Konjugate steigern.

Demgemäß umfaßt die vorliegende Erfindung ebenfalls Verfahren zur Herstellung eines Arznei- oder Diagnosemittels, bei denen man ein HAS-Wirkstoff-Konjugat nach einem der obigen Verfahren herstellt und mit einem der im Stand der Technik bekannten, pharmazeutisch verträglichen Träger, Adjuvans oder Hilfsstoff vermischt, sowie nach diesem Verfahren erhältliche Arznei- oder Diagnosemittel.

Die medizinische Verwendung des entsprechenden Arzneimittels hängt von der Art des Wirkstoffs ab. Wird beispielsweise Hämoglobin als Wirkstoff eingesetzt, kann das Konjugat als Sauerstoff-Transport-Mittel verwendet werden. Wird andererseits ein Zytokin als Wirkstoff für die Herstellung verwendet, kann das Konjugat beispielsweise in der Tumortherapie verwendet werden. Die Konzentration des jeweils zu verwendenden Konjugates in dem Arzneimittel kann von jedem durchschnittlichen Fachmann ohne weiteres in Dosis-Wirkungstests ermittelt werden.

Die Diagnosemittel können in vivo oder in vitro zur Diagnose von Erkrankungen oder Störungen verwendet werden. Wenn als Wirkstoff ein Antikörper oder Antikörperfragment eingesetzt wird, eignet sich das Konjugat beispielsweise für die Durchführung der im Stand der Technik üblichen ELISA-Nachweisverfahren.

In den Beispielen wurden die nachfolgend beschriebenen Materialien verwendet:

**Humanes Serumalbumin: Sigma-Aldrich A3782**

| | |
|---|---|
| HES 130kD: | Typ 130/0,5, hergestellt aus T91SEP (Fresenius Kabi) |
| Daten: | M_{w}: 130 000 ± 20 000D |
| Mₙ: | 42 600 D |
| | |
| HES 10 kD: | Typ HHH 4-2 (Fresenius Kabi) |
| Daten: | M_{w}: 9 800 D |
| Mₙ: | 3 695 D |
| | |
| EDC: | Sigma-Aldrich Nr. 16.146-2 |
| (Ethyldimethyl-aminopropyl-Carbodiimid) | |
| HOBt | Sigma-Aldrich Nr. 15.726-0 |
| (1-Hydroxy-1H-Benzotriazolhydrat) | |
| | |
| DIG-Glykandetektionskit: | Roche-Boehringer Nr. 1142 372 |

Die folgenden Beispiele 1 bis 6 beschreiben die Kopplung von HSA und Diaminobutan an HES mit oxidierten reduzierenden Endgruppen oder die direkte Kopplung von HSA an HES. HSA und Diaminobutan sind dabei lediglich Beispiele für die oben definierten Wirkstoffe. Beispiel 7 beschreibt die Kopplung von Oligonukleotiden an HES.

### Beispiel 1: Selektive Oxidierung der reduzierenden Endgruppen der Hydroxyethylstärke:

Für die selektive Oxidierung der reduzierenden Endgruppen der Hydroxyethylstärke (130 kD und 10 kD) wurde diese in einer minimalen Menge Wasser aufgelöst und mit einer verschiedenen Menge einer Iod-Lösung und einer KOH-Lösung versetzt.

Die Mischung wurde gerührt, bis die auf I₂ hinweisende Farbe verschwand. Diese Vorgehensweise wurde mehrfach wiederholt, um die Zugabe einer größeren Menge der Iod-Lösung und KOH-Lösung zu erreichen. Die Lösung wurde anschließend über einen Amberlite IR 120 Na⁺ Kationenaustauscherharz gereinigt, für 20 Stunden gegen destilliertes Wasser dialysiert (Dialyseröhrchen mit einer Ausschlussgrenze von 4-6 kD) und lyophylisiert.

Der Grad der Oxidation wurde jeweils mittels des in Somogyi, N. (Method in Carbohydride Chemistry, Vol. 1, (1962) S. 384-386) offenbarten Verfahrens ermittelt. Die Protokolle der Oxidationsreaktion sind in Tabelle 1 wiedergegeben.

**Tabelle 1: Oxidation der reduzierenden Endgruppen der HES (130 kD und 10 kD) unter verschieden Bedingungen**

| Verfahren | HES (Mn) | Iod-Lösg. 0.1N | KOH-Lösg. 0.1M | Lösungs mittel | Reaktionszeit | Ertrag |
|---|---|---|---|---|---|---|
| OXIDATION (1) | 1 g | 0.3 ml . | 0.5 ml | Wasser | 4 h | 30.1% |
| HES 130 | 2.4x10⁻⁵mol | 3.0x10⁻⁵mol | 5.0x10⁻⁵ mol | 4.0 ml | 25°C | |
| OXIDATION (2) | 4g | 1.0ml | 1.5 ml | Wasser | 0. Nacht | 24.8% |
| HES 130 | 9.4x0⁻⁵mol | 1.0x10⁻⁴mol | 1.5x10⁻⁴ mol | 6.0 ml | 25°C | |
| OXIDATION (3) | 5 g | 1.2 ml | 1.5 ml | Wasser | ü. Nacht | 24.3% |
| HES 130 | 1.2x10⁻⁴ mol | 1.2x10⁻⁴ mol | 1.5x10⁻⁴ mol | 7.5 ml | 25°C | |
| OXIDATION (4) | 5 g | 3.0 ml | 4.5 ml | Wasser | 0. Nacht | 60.8% |
| HES 130 | 1.2x10⁻⁴mol | 3.0x10⁻⁴mol | 4.5x10⁻⁴ mol | 7.5 ml | 25°C | |
| OXIDATION (5) | 5 g | 4.0 ml | 5 ml | Wasser | 0. Nacht | 80,0% |
| HES 130 | 1.2x10⁻⁴mol | 4.0x10⁻⁴mol | 5.0x10⁻⁴ mol | 7.5 ml | 25°C | |
| OXIDATION (6) | 8 g | 7.0 ml | 11.5 ml | Wasser | 0. Nacht | 88.4% |
| HES 130 | 1.9x10⁻⁴mol | 7.0x10⁻⁴mol | 1.2x10⁻³ mol | 7.5 ml | 25°C | |
| OXIDATION (7) | 10 g | 10 ml | 20 ml | Wasser | 0. Nacht | 100% |
| HES 130 | 2.4x10⁻⁴mol | 1.0x10⁻³mol | 2.0x10⁻³ mol | 7.5 ml | 25°C | |
| OXIDATION (1) | 5 g | 2.0 ml | 2.0 ml | Wasser | 20 h | 3.0% |
| HES 10 | 1.4x10⁻³mol | 2.0x10⁻⁴mol | 2.0x10⁻⁴ mol | 10.0 ml | 25°C | |
| OXIDATION (2) | 5 g | 3.5 ml | 4.5 ml | Wasser | 0. Nacht | 5.3% |
| HES 10 | 1.4x10⁻³mol | 3.5x10⁻⁴mol | 4.5x10⁻⁴ mol | 10.0 ml | 25°C | |
| OXIDATION (3) | 15 g | 21.0 ml | 31.0 ml | Wasser | 0. Nacht | 10.5% |
| HES 10 | 4.1x10⁻³mol | 2.1x10⁻³mol | 3.1x10⁻³ mol | | 25°C | |
| OXIDATION (4) | 8 g | 83.0 ml | 180.0 ml | Wasser | 0. Nacht | 80.0% |
| HES 10 | 2.2x10⁻³mol | 8.3x10⁻³mol | 1.8x10 mol | | 25°C | |
| OXIDATION (5) | 7 g | 95.0 ml | 210.0 ml | Wasser | 0. Nacht | 100,0% |
| HES 10 | 1.9x10⁻³mol | 9.5x10⁻³mol | 2.1x10⁻² mol | | 25°C | |
| OXIDATION (6) | 6.4 g | 50 ml | 150 ml | Wasser | 0. Nacht | 100.0% |
| HES 10 | 1.7x10⁻³mol | 5.0x10⁻³ | 1,5x10⁻² | | 25°C | |

Die in dieser Tabelle zusammengefaßten Protokolle werden nachfolgend für die Oxidation (6), HES 10 kD, im Detail wiedergegeben: 6.4 g HES 10kD (1.7 mmol) wurden in einem Reaktionsgefäß unter ständigem Rühren in wenigen ml Wasser gelöst. Dazu wurden 50 ml einer 0.1 N Jodlösung (5.0 mmol) und 150 ml einer 0.1 N KOH-Lösung (15 mmol) gegeben. Diese Mischung wurde über Nacht bei 25°C stehen gelassen. Der Mix wurde mit Amberlite IR 120 (Na+-Form) gereinigt und gegen Wasser dialysiert (Dialyseschlauch Celluloseacetat; Cut-off 4 bis 6 kD). Das dialysierte Produkt wurde lyophilisiert (Heraeus-Christ Alpha, Kolbentrocknung über Nacht).

Wie Tabelle 1 zu entnehmen ist, wurde eine vollständige Oxidation der reduzierenden Endgruppen (entspricht Ertrag 100%) der HES 130 kD erzielt, nachdem die Iod-Menge von 3,0 x 10⁻⁵ mol bis auf 1,0 × 10⁻³ mol erhöht wurde.

Für eine vollständige Oxidation der reduzierenden Endgruppen der HES 10 kD war eine weitere Steigerung der Iod-Menge auf eine Konzentration von mehr als 2,1 x 10⁻³ mol notwendig.

### Beispiel 2: Bindung von HES mit oxidierten reduzierenden Endgruppen an HSA in wässeriger Phase:

Für die Kopplung wurde Hydroxyethylstärke mit oxidierten reduzierenden Endgruppen (ox-HES) und HSA vollständig in Wasser gelöst. Als die Lösung klar war, wurde Ethyldimethyl-aminopropyl-Carbodiimid (EDC) in Wasser gelöst zugegeben. Nach Aktivierung durch EDC bei gemäßigtem Rühren wurden weitere Mengen an EDC zugegeben. Die Reaktion wurde gegebenenfalls mit HOBt aktiviert und über Nacht stehengelassen. Das Produkt wurde durch Dialyse gegen destilliertes Wasser für 15 Stunden gereinigt und anschließend lyophylisiert (nachfolgend als Verfahren A bezeichnet).

Die Protokolle der Kopplungsreaktion finden sich in Tabelle 2.

**Tabelle 2: Kopplungsreaktionen zwischen HES (130 kD und 10 kD) mit oxidierten reduzierenden Endgruppen und HSA unter verschiedenen Bedingungen (Verfahren A; Zahl in der Klammer in Spalte HES gibt das Oxidationsverfahren nach Tabelle 1 wieder)**

| Verfahren A | HSA | ox-HES (Mn) | EDC | HOBt | Lösung mittel | Akti vier. | Reaktion |
|---|---|---|---|---|---|---|---|
| Kopplung I | 300 mg | 100 mg (1) | 25 | 100 mg | H₂O/Dioxan | 1.5 h | 4 h |
| ox-HES 130 | 4.4x10⁻⁶mol | 2.4x10⁻⁶mol | mg 1.6x10⁻⁴mol | 7.7x10⁻⁴ mol | 13 ml/2 ml | 3-4°C | 25°C |
| Kopplung II | 100 mg | 300 mg (2) | 15 mg | 100 mg | H₂O/Dioxan | 1.5 h | 0. Nacht |
| ox-HES 130 | 1.5x10⁻⁶ mol | 7.0x10⁻⁶mol | 9.7x10⁻⁵mol | 7.7x10⁻⁴mol | 10 ml/3 ml | 3-4°C | 25°C |
| KopplungIII ox-HES | 200 mg | 3.8 g (5) | 46.5 mg | 20 mg | H₂O/Dioxan | 0 h | 24 h |
| 130 | 3.0x10⁻⁶mol | 8.9x10⁻⁵mol | 3.0x10⁻⁴ mol | 1.5x10⁻⁴mol | 10 ml/3 ml | | 25°C |
| Kopplung IV | 100 mg | 1.9 (5) | 25 mg | 20 mg | Wasser | 1.5 h | 0. Nacht |
| ox-HES 130 | 1.5x10⁻⁶mol | 4.5x10⁻⁵ mol | 1.6x10⁻⁴mol | 1.5x10⁻⁴mol | | 3-4°C | 25°C |
| Kopplung V | 200 mg | 4.3 g (5) | 100 mg | 0 mg | Wasser | 0 h | 0. Nacht |
| ox-HES 130 | 3.0x10⁻⁶mol | 1.0x10⁻⁴mol | 6.0x10⁻⁴mol | | | | 25°C |
| Kopplung VI | 100 mg | 130 mg (7) | 50 mg | 0 mg | Wasser | 0 h | 5 h |
| ox-HES 130 | 1.5x10⁻⁶ | 3.0x10⁻⁶mol | 3.0x10⁻⁴mol | | 5ml + 10ml | | 25°C |
| KopptungVII | 100 mg | 130 mg (7) | 200 mg | 0 mg | Wasser* 5ml | 0 h | 24 h |
| ox-HES 10 | 1.5x10⁻⁶ | 3.0x10⁻⁶mol | 3.0x10⁻⁴mol | | + 2x 10ml | | 25°C |
| Kopplung I | 100 mg | 300 mg (1) | 5 mg | 100 mg | H₂O/Dioxan | 1.5 h | 0. Nacht |
| ox-HES 10 | 1.5x10⁻⁶mol | 8.1x10⁻⁶mol | 3.0x10⁻⁵mol | 7.7x10⁻⁴mol | 13 ml/2 ml | 3-4°C | 25°C |
| Kopplung II | 70 mg | 1.0 g (2) | 15.5 mg | 0 mg | Wasser | 10 ml | 0. Nacht |
| ox-HES 10 | 1.0x10⁻⁶mol | 2.7x10⁻⁴mol | 1.0x10⁻⁴ mol | | | 0 h | 25°C |
| KopplungIII | 200 mg | 3.0 g (3) | 77.5 mg | 20 mg | Wasser | 0 h | 6 h |
| ox-HES 10 | 3.0x10⁻⁶mol | 8.1x10⁻⁴mol | 5.0x10 mol | 1.5x10-4mol | | | 25°C |
| Kopplung IV | 50 mg | 7.4 g (4) | 282 mg | 0 mg | Wasser | 0 h | 0. Nacht |
| ox-HES 10 | 7.4x10⁻⁷mol | 2.0x10⁻³mol | 1.5x10⁻³mol | | | | 25°C |
| Kopplung V | 100 mg | 103 g (6) | 93 mg | 0 mg | Wasser | 0 h | 20 h |
| ox-HES 10 | 1.5x10⁻⁶mol | 2.8x10⁻⁵mol | 5.6x10⁻⁴ mol | | 4 ml | | 25°C |
| Kopplung VI | 100 mg | 103 g (6) | 200 mg | 0 mg | Wasser* | 0 h | 30 h |
| ox-HES 10 | 1.5x10⁻⁶m0l | 2.8x10⁻⁵mol | 1.2x10⁻³mol | | 3x5 ml | | 25 °C |

| | | | | | | | |
|---|---|---|---|---|---|---|---|
| - Zugabe der EDC-Lösung mit einem Tropftrichter innerhalb von 60 Min. | | | | | | | |

Die Kopplungsreaktion VII zwischen ox-HES 130 kD und HSA wird im folgenden im Detail erläutet: In einem Rundkolben wurden 130 mg ox-HES 130 kD (Oxidationsgrad ca. 100%) und 100 mg HSA unter Rühren in ca. 5 ml Wasser bei Raumtemperatur gelöst. Sobald die Lösung klar war, wurden 200 mg EDC in 3 Portionen jeweils in 5-10 ml Wasser gelöst über einen Zeitraum von einer Stunde zugetropft. Zwischen jeder Zugabe ließ man ca. 4 h bei Raumtemperatur rühren. Nach 24 h Reaktionszeit wurde das Gemisch gegen Wasser dialysiert (Dialyseschlauch Celluloseacetat; Cutoff 4-6 kD). Anschließend wurde das Produkt lyophilisiert.

### Beispiel 3: Direkte Bindung von HES an HSA in wässriger Phase:

Das Prinzip dieser Reaktion basiert auf der Bildung von Schiff'schen Basen zwischen HES und Aminogruppen des Proteins, wobei die Reaktion durch die Reaktion der Schiff'schen Base zu dem entsprechenden Amin mittels NaBH₄ gesteuert wird (nachfolgend als Verfahren B bezeichnet).

Dafür wurde HES vollständig in wenig Wasser gelöst. Dazu wurde in Boratpuffer, pH 9.0, gelöstes HSA gegeben. Zu dieser Lösung wurde NaBH₄ gegeben und bei Raumtemperatur unter Rühren belassen. Ein weiteres Aliquot von HES 130 kD, gefolgt von weiterem NaBH₄ wurden zugegeben. Nach Abschluß der Reaktion wurde wie beschrieben dialysiert und gefriergetrocknet.

Die Protokolle der einzelnen Versuche sind in Tabelle 3 zusammengefaßt.

**Tabelle 3: Direkte Kopplung zwischen HES (130 kD und 10 kD) und HSA unter verschiedenen Bedingungen (Verfahren B)**

| Verfahren B | HSA | HES (Mn) | NaBH4 | Puffer pH | Reaktionszeit |
|---|---|---|---|---|---|
| Kopplung I | 50 mg | 500 mg | 500 mg | Na₂HPO₄, 0 ml | 48 h |
| HES 130 | 7.5x10⁻⁷mol | 1.2x10⁻⁵mol | 1.3x10⁻²mol | 7.4 | 25°C |
| Kopplung II | 100 mg | 1.0 g | 60 mg | Na₂HPO₄ 1 ml | 20 h |
| HES 130 | 1.5x10⁻⁶mol | 2.4x10⁻⁵mol | 1.6x10⁻³mol | 7.4 | 25°C |
| Kopplung III | 50 mg | 9.8 g | 285 mg | Na₂HPO₄. 1 ml | 36 h |
| HES 130 | 7.5x10⁻⁷mol | 2.3x10⁻⁴mol | 7.5x10⁻³mol | 7.4 | 25°C |
| Kopplung IV | 50 mg | 2.0 | 180 mg | Borat 0.1M | 30 h |
| HES 130 | 7.5x10⁻⁷mol | 4.7x10⁻⁵mol | 4.7x10⁻³mol | 9.0 | 25°C |
| Kopplung V | 50 mg | 4.0 g | 60 mg | Borat 0.1M | 100 h |
| HES 130 | 7.5x10⁻⁷mol | 9.4x10⁻⁵mol | 1.6x10⁻³mol | 9.0 | 25 °C |
| Kopplung | 50 mg | 2.8 g | 28 mg | Borat 0.1M | 80 h |
| HES 10 | 7.5x10⁻⁷mol | 9.4x10⁻⁵mol | 1.6x10⁻³mol | 9.0 | 25°C |

Im Einzelnen wurden für die Kopplung der HES 130 kD 2.0 g dieser Verbindung vollständig in Wasser gelöst (ca. 5 ml). Dazu wurden 50 mg in 1 ml 0.1 M Boratpuffer, pH 9,0, gelöstes HSA gegeben. Zu dieser Lösung wurden 30 mg NaBH₄ gegeben und unter Rühren bei Raumtemperatur belassen. Nach 18 h wurde ein weiteres Aliquot von 2.0 g HES 130 kD, gefolgt von weiteren 30 mg NaBH₄ zugegeben. Nach insgesamt 100 h Reaktionszeit wurde dialysiert und gefriergetrocknet (Kopplung V, HES 130 kD).

Für die Kopplung der HES 10 kD wurden 1.4 g dieser Verbindung komplett in Wasser gelöst (ca. 5 ml). Dazu wurden 50 mg in 1 ml 0,1 M Boratpuffer, pH 9, 0, gelöstes HSA gegeben. Zu dieser Lösung wurden 14 mg NaBH₄ gegeben und unter Rühren bei Raumtemperatur belassen. Nach 18 h wurde ein weiteres Aliquot von 1.4 g HES 10 kD, gefolgt von weiteren 14 mg NaBH₄ zugegeben. Nach insgesamt 80 h Reaktionszeit wurde dialyisiert und gefriergetrocknet (Kopplung I, HES 10 kD).

### Beispiel 4 Analyse der Kopplungsprodukte mittels GPC:

Die Reaktionsprodukte wurden zunächst unter Verwendung von Gel-Permeations-Chromatographie (GPC) analysiert.
4.1 Für die GPC wurde ein FPLC-Gerät (Pharmacia), welches mit einem HPLC UV Monitor (Hitachi) verbunden war, verwendet. Ferner wurden die folgenden Bedingungen verwendet:

| | |
|---|---|
| Säule: | Superose 12 HR 10/30 (1x30 cm) (Pharmacia) |
| W-Monitor: | 280 nm |
| Pumpe: | 0,2 ml/min |
| Puffer: | 50 mM Phosphat/150 mM NaCl pH 7,2. |

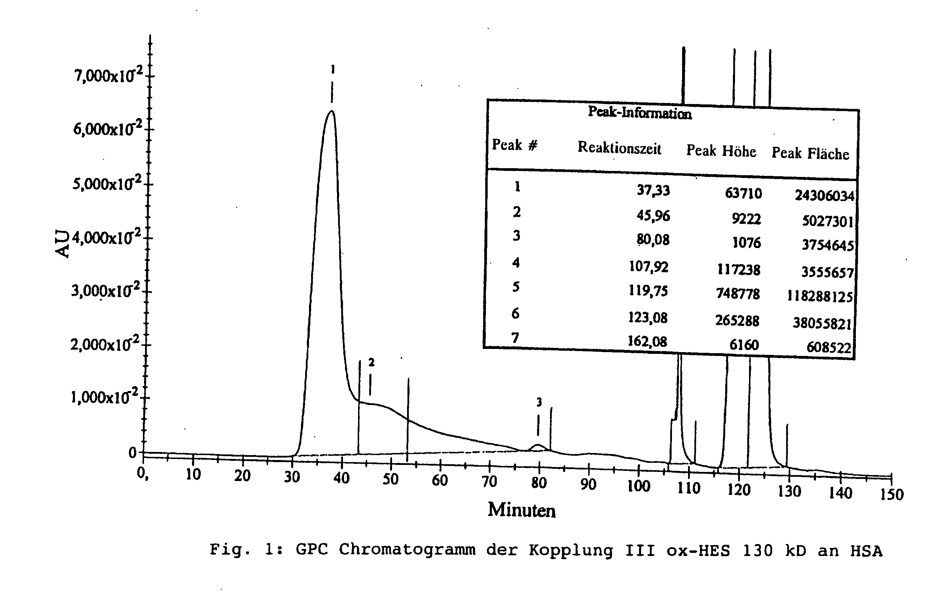

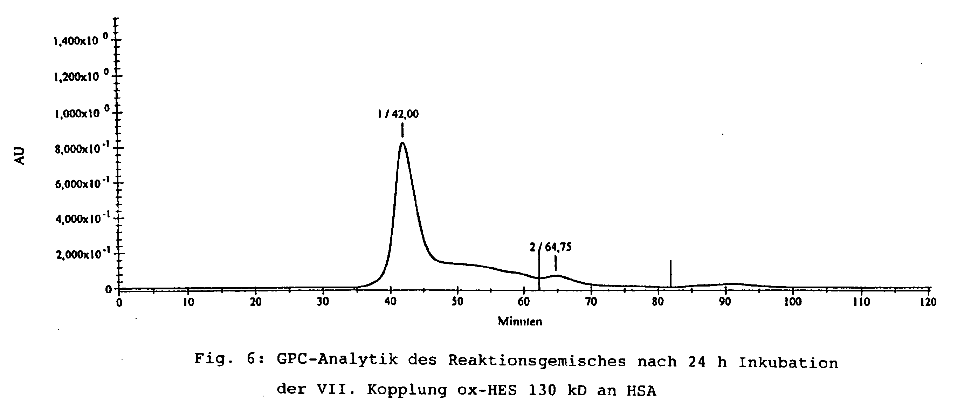

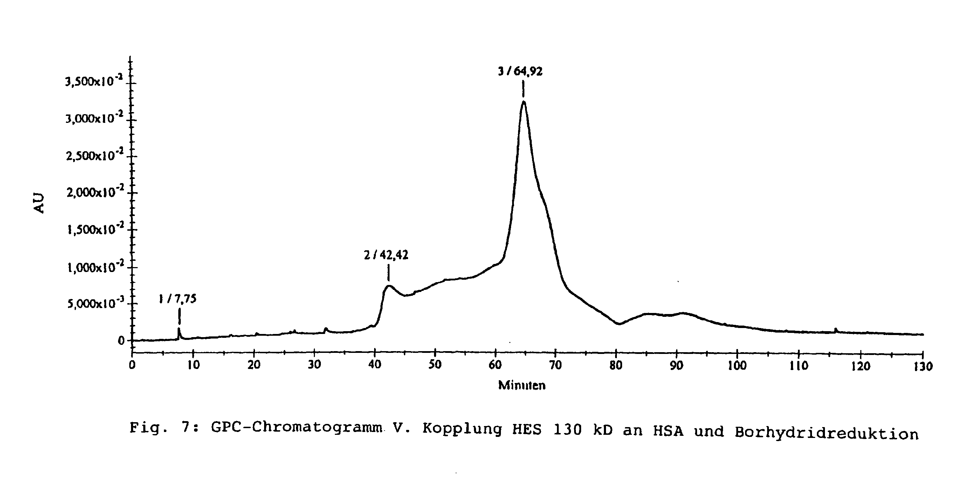

Unter diesen Bedingungen wird der HSA-Peak üblicherweise nach 63 min gefunden, wobei zusätzlich ein kleiner Peak bei etwa 57 min gemessen werden kann, der durch HSA-Dimere verursacht wird. Die mittels GPC erhaltenen Chromatogramme lassen sich wie folgt analysieren:
4.2 Fig. 1 ist ein Chromatogramm, das die Größenverteilung der Produkte nach Kopplung von ox-HES 130 kD an HSA (Kopplung III) zeigt. Bei diesem Kopplungsverfahren wurden ohne HOBt-Aktivierung sehr gute Ergebnisse erzielt. Ein deutlicher, einzelner breiter Peak wurde bei 37 Min. und eine weitere, kleinere Bande bei 45 Min. gemessen, was auf ein Kopplungsprodukt mit höherem Molekulargewicht als HSA hinweist. Gleichzeitig wurden Spuren von nicht-modifiziertem HSA gefunden.

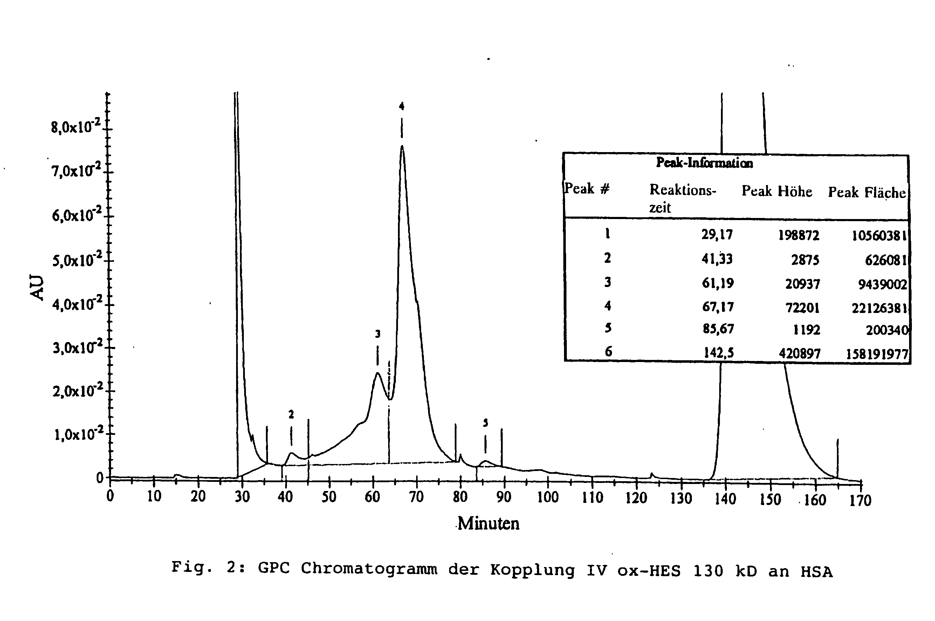

Fig. 2 zeigt die Größenverteilung der Produkte nach Kopplung von ox-HES 130 kD an HSA (Kopplung IV). Die Reaktion wurde mit HOBt aktiviert. Es zeigt sich, dass diese Aktivierung die Ausbeute verringert, möglicherweise durch Förderung von Nebenreaktionen.

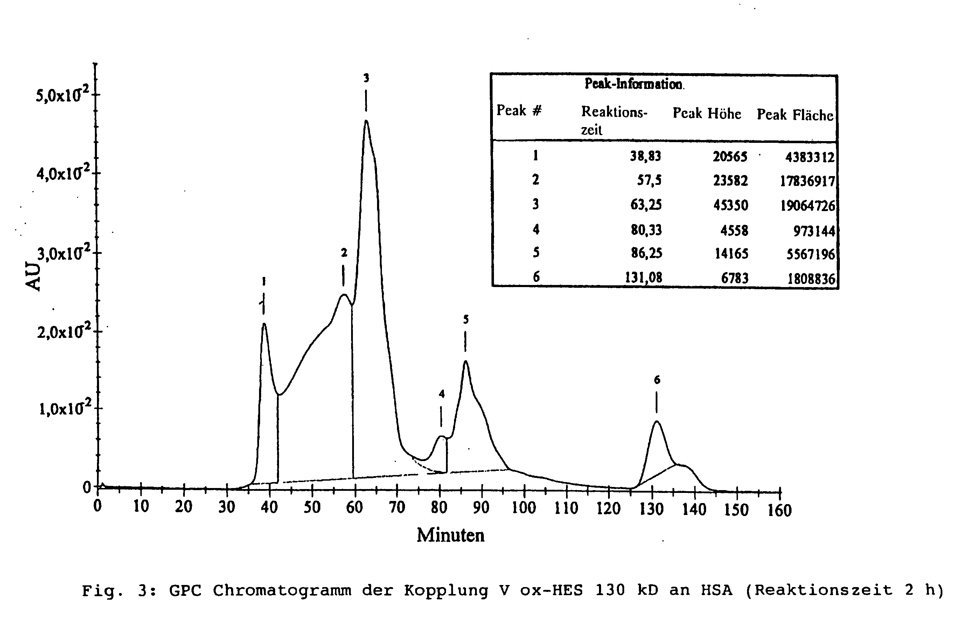

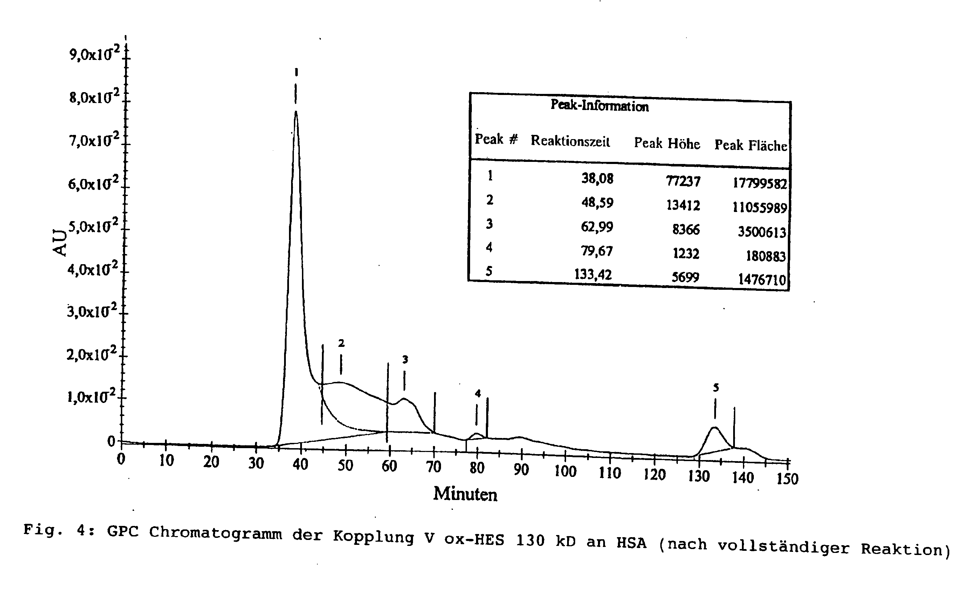

Die Fig. 3 und 4 zeigen die Größenverteilung der Reaktionsprodukte während und nach der Kopplungsreaktion von ox-HES 130 kD an HSA (Kopplung V). Nach 2 Stunden Reaktionszeit wurde nicht-modifiziertes HSA als Produkt mit der höchsten Konzentration, daneben aber auch erste Kopplungsprodukte mit höherem Molekulargewicht gefunden. Nach Ablauf der Reaktion wurde ein homogenes Kopplungsprodukt mit einer Retentionszeit von ca. 35 Min. in hoher Konzentration gefunden. Nicht-modifiziertes HSA und andere Kopplungsprodukte lagen in relativ geringer Konzentration vor.

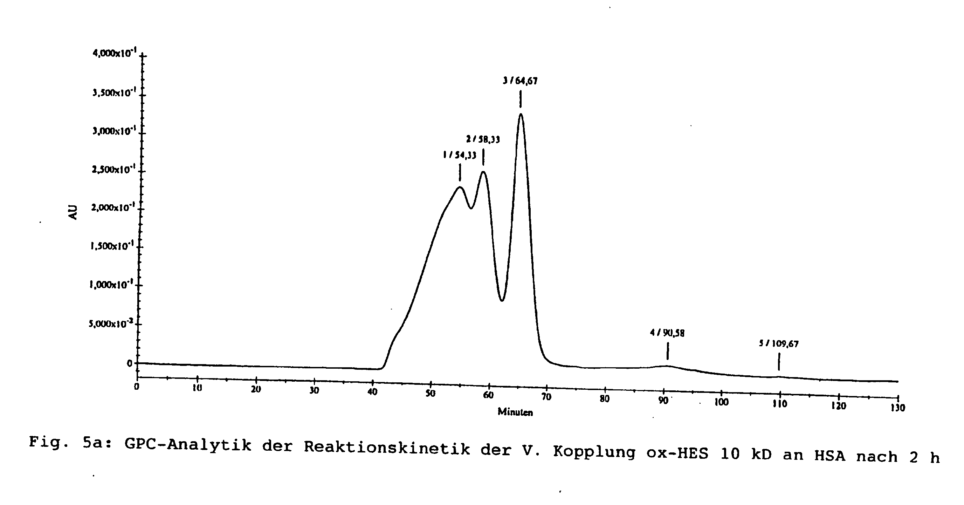

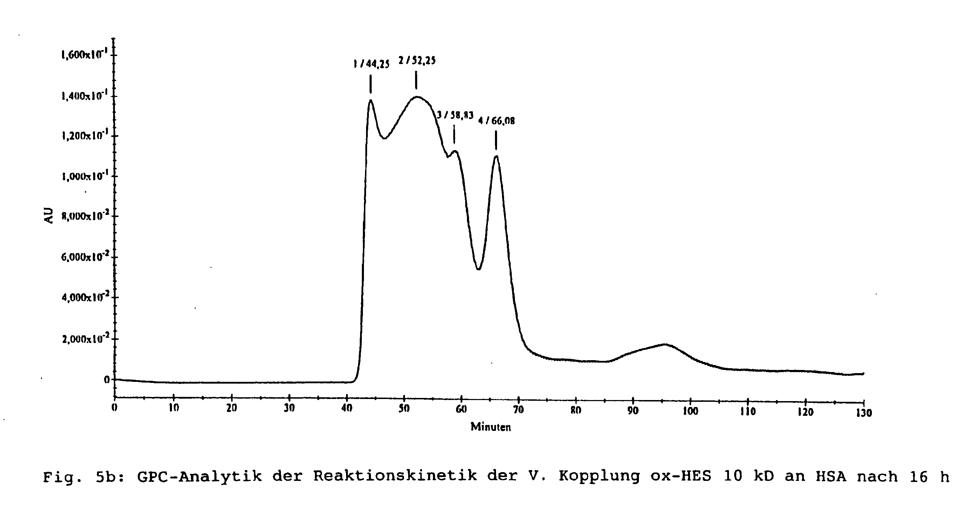

Fig. 5 zeigt die entsprechende Größenverteilung der Reaktionsprodukte während und nach der Kopplungsreaktion von ox-HES 10 kD an HSA (Kopplung V). Auch hier zeigt sich, dass die Konzentration der Kopplungsprodukte mit einem Molekulargewicht, das oberhalb des Gewichtes von HSA liegt, im Verlauf der Reaktion zunimmt.
Eine Kopplungsreaktion, bei der nahezu alle HSA-Moleküle in ein homogenes Kopplungsprodukt überführt werden konnten, wird schließlich in Fig. 6 gezeigt (Reaktionsprodukte der Kopplung VII).
4.2 Ein Beispiel für die Chromatogramme, die bei der Analyse der direkten Kopplung von HES an HSA erhalten wurden, wird in Fig. 7 gezeigt (Verfahren B, HES 130 kD, Kopplung V). Ein deutlicher Peak bei ca. 65 Min. (HSA) ist zu erkennen. Darüber hinaus wurde aber auch ein Kopplungsprodukt nachgewiesen (Peak bei ca. 42 Min.).

### Beispiel 5 Analyse der Kopplungsprodukte mittels SDS-PAGE und Western Blot:

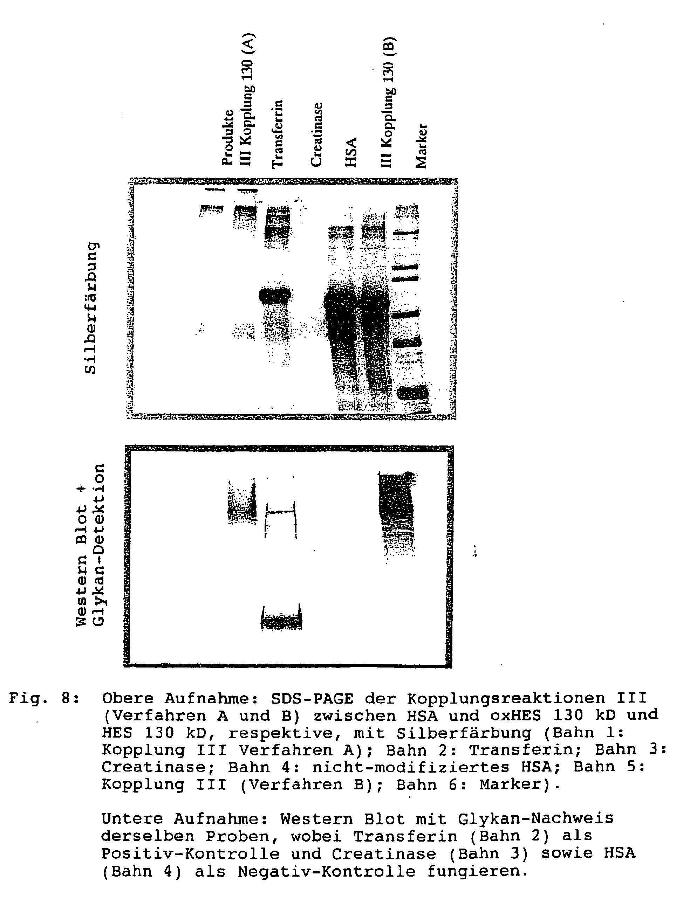

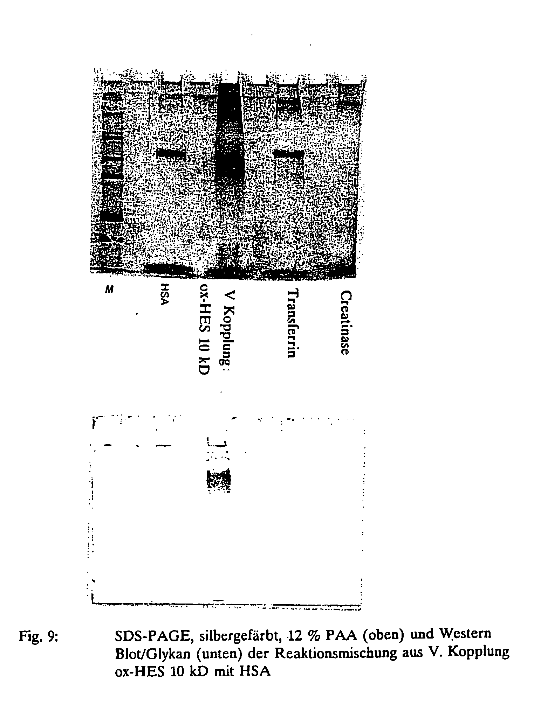

5.1 PAGE wurde in Gegenwart von Natriumdodecylsulfat (SDS) unter Verwendung eines Miniprotean II Gerätes der Firma Biorad und 7,5 % Acrylamidgele durchgeführt. Die Durchführung der Elektrophorese erfolgte nach den Vorschriften des Herstellers. Die Gele wurden nach dem Verfahren von Blum (Elektrophoresis, Vol. 8, (1997) S. 93-99) mit Silber gefärbt, um Proteine sichtbar zu machen.
   Die Gegenwart von Glykanen in den Kopplungsprodukten wurde mittels Western-Blot und Glykan-Detektions-Kit der Firma Roche-Boehringer nachgewiesen. Nach Auftrennung der Produkte mittels SDS-PAGE wurden diese unter Verwendung des Blotting-Gerätes der Miniprotean II Elektrophoreseeinheit auf eine Nitrocellulosemembran überführt. Die Membran wurde anschließend mittels Periodat unter Bedingungen oxidiert, bei denen lediglich die vicinalen OH-Gruppe oxidiert werden. Diese wurden anschließend mit einem Amino-funktionalisierten Digoxygenin umgesetzt. Gebundenes Digoxygenin wurde mittels spezifischer monoklonaler Antikörper, welche an eine alkaline Phosphatase gebunden waren, nachgewiesen. Dafür wurde ein Substrat der Phosphatase (4-Nitro-tetrazoliumchlorid/5-Brom-4-chlor-3-indolylphosphat) zugegeben, welches ein schwerlösliches blau-violettes Produkt erzeugt. Dieses Produkt schlägt sich auf der Membran nieder und lässt somit die Banden sichtbar werden. Nicht-modifiziertes HSA und Kreatinase wurden als Negativkontrollen eingesetzt, während Transferrin als Positivkontrolle fungierte.
   Die genauen Verfahrensschritte sind im Beipackzettel zu diesem Kit beschrieben (Roche-Boehringer).
5.2 Die Fig. 8 und 9 zeigen jeweils eine Aufnahme des Silbergefärbten SDS-PAGE-Gels (oben) und die Aufnahme der entsprechenden Produkte nach Übertragung auf eine Membran und Glykannachweis (unten). Wie sich aus diesen Figuren entnehmen läßt, entsteht während der Kopplungsreaktion ein relativ homogenes Glykan als Reaktionsprodukt, während gleichzeitig die Konzentration an nicht-modifiziertem HSA abnimmt.

### Beispiel 6 Abklärung möglicher Nebenreaktionen:

Zur Klärung, ob Nebenreaktionen in Form einer Selbstkondensation von HES mit oxidierten reduzierenden Endgruppen erfolgen, wurden folgende Reaktionsansätze erstellt:

**Tabelle 4: Reaktionsansätze zur Abklärung von Nebenreaktionen.**

| | ox-HES | EDC | HOBt | Wasser | Reaktionszeit |
|---|---|---|---|---|---|
| HES 130 kD | 500 mg | 15 mg | - | 5.0 ml | 30 h |
| | 1.2x10⁻⁵mol | 7.8x10⁻⁵ mol | | | 25 °C |
| HES 130 kD | 500 mg | 15 mg | gesättigte | 5.0 ml | 30 h |
| | 1.2x10⁻⁵mol | 7.8x10⁻⁵ mol | Lösung | | 25°C |
| HE5 10 kD | 100 mg | 3.4 mg | - | 5.0 ml | 30 h |
| | 2.7x10⁻⁵mol | 1.8x10⁻⁵ mol | | | 25°C |
| HES 10 kD | 100 mg | 3.4 mg | gesättigte | 5.0 ml | 30 h |
| | 2.7x10⁻⁵mol | 1.8x10⁻⁵ mol | Lösung | | 25°C |
| HES 130 kD | 700 mg | 31 mg | - | 5.0 ml | 30 h |
| | 1.6x10⁻⁵mol | 1.6x10⁻⁴ mol | | | 25 °C |
| HES 130 kD | 700 mg | 31 mg | gesättigte | 5.0 ml | 30 h |
| | 1.6x10⁻⁵mol | 1.6x10⁻⁴ mol | Lösung | | 25°C |

Ziel der Experimente war es, nachzuweisen, inwieweit eine mögliche Selbstkondensation von HES in Gegenwart oder Abwesenheit von HOBt stattfindet. Die Proben, wurden lyophilisiert und analysiert.

Mittels GPC und Streulichtmessungen konnten innerhalb der Detektionsgrenzen von einigen Prozent keine Hinweise auf Molekulargewichtsvergrößerungen gefunden werden.

### Beispiel 7: Kopplung von oxidierter HES an DNA und Analyse der Funktionalität der Kopplungsprodukte

### (Kopplung mit nicht erfindungsgemäßen Wirkstoff)

### Reaktionsprinzip:

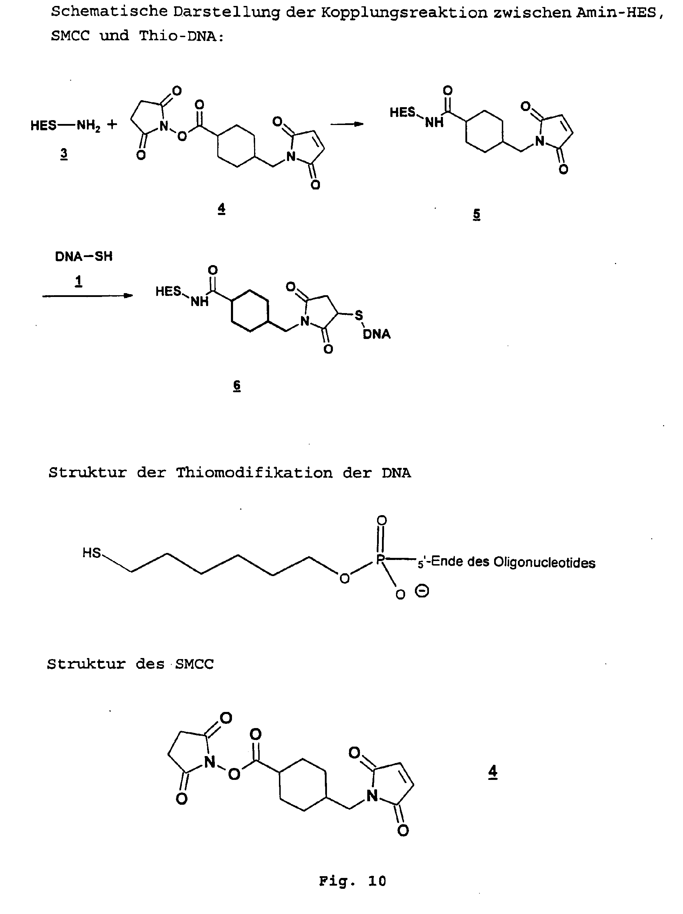

Eine schematische Darstellung der Reaktion findet sich in Fig. 10. Im ersten Schritt reagiert die Aminogruppe der Amin-HES12KD **3** mit der N-Hydroxysuccinimid-gruppe des SMCC **4** zum Konjugat **5.** Durchs Zentrifugieren mit dem Zentrifugen-Dialyseeinsatz wird nicht reagiertes SMCC **4** abgetrennt. Die Maleimid-Gruppe des Konjugats **5** reagiert dann mit der Thiol-Gruppe der Thio-DNA **1** zum dem gewünschten Produkt **6.** Der in **6** fett markierte Bereich ist nur ein Spacer und kann jede Gestalt haben.

Die Überprüfung der biologischen Aktivität des Konjugates **6**. erfolgte über die Spaltung durch das Restriktionsenzym EcoR1. Ein Restriktionsenzym schneidet nur doppelsträngige DNA mit intakter Erkennungssequenz.

### DNA:

Verwendet wurde doppelsträngige DNA, synthetisiert von MWG Biotech AG, Ebersberg. Die Sequenzen der Einzelstränge lauten:

| | |
|---|---|
| SEQ ID NO. 1: | 5' - GTAGAGACAGGAGGCAGCAGTTGAATTCGCAGGGT- GAGTAGCAGTAGAGC-3' ; |
| SEQ ID NO. 2: | 5'- G C T C T A C T G C T A C T C A C C C T G C G A A T T - CAACTGCTGCCTCCTGTCTCTAC-3' ; |

modifiziert mit 5'Thiol C6 S-S durch MWG (vgl. Fig.10).

Die beiden DNA Einzelstränge wurden in bidest. Wasser in einer Konzentration von je 2 µg/µl gelöst und im Verhältnis 1 : 1 bei 96°C zu einer doppelsträngigen Thio-DNA **1** mit einer Konzentration von 2 µg/µL hybridisiert.

### Analyse der Produkte:

Die Analyse erfolgte durch Gelelektrophorese in einem 4% Agarose-Gel mit einem TBE-Laufpuffer aus 45 mM Tris-Borat, 1mM EDTA, pH 8.0 von je 1 µg DNA in Gegenwart von 50 µg Ethidiumbromid pro 100 ml Gel. Die Fotos wurden aufgenommen mit einem CCD-System Modular (INTAS Imaging Instruments, Göttingen, D) und einem UV-Transilluminator UVT-20 S/M/L (Herolab GmbH, Wiesloch, D) bei 312 nm.

Vom Reaktionsgemisch wurden 1 µL (1µg DNA) abgenommen und geschnitten mit 1 µL (20 U) EcoR1- Restriktionsenzym (New England Biolabs GmbH, Schwalbach/Taunus, D), 1 µL Reaktionspuffer (50 mM Natriumchlorid, 100 mM Tris-HCl, 10 mM Magnesiumchlorid, 0,025% Triton X-100, pH 7.5 von New England Biolabs) und 7µL bidest. Wasser bei 37°C für 3h.

### Modifikation der HES

Die Oxidation von HES12KD (Fresenius, Charge 2540SR2.5P) mit einem mittleren Molgewicht von 12000 g/mol mit Iod-LÖsung zum Oxo-HES12KD **2** wurde nach den in DE 19628705 offenbarten Verfahren durchgeführt.

### Reaktion von 1,4-Diaminobutan mit Oxo-HES12KD 2:

1,44 g (0,12 mmol) des Oxo-HES12KD **2** wurden in ml wasserfreiem Dimethylsulfoxid (DMSO) gelöst, unter Stickstoff zu einer Lösung aus 1.5 ml (1, 50 mmol)-1,4 Diaminobutan getropft und bei 40°C für 19 h gerührt. Das Reaktionsgemisch wurde zu einem Gemisch aus 80 ml Ethanol und 80 ml Aceton gegeben. Der gebildete Niederschlag wurde abzentrifugiert und in 40 ml Wasser aufgenommen. Die Lösung wurde für 4 Tage gegen Wasser dialysiert (SnakeSkin Dialyseschlauch, 3.5 KD cut off, Perbio Science Deutschland GmbH, Bonn, D) und anschließend gefriergetrocknet. Die Ausbeute betrug 80% (1.06 g) Amino-HES12KD **3**.

### Kopplung von Amino-HES12KD 3 an Thio-DNA 1.

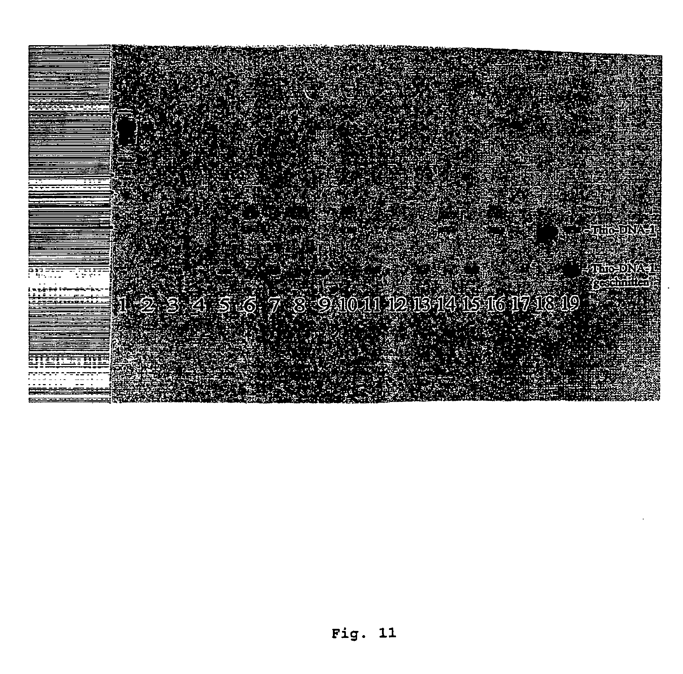

Zu 400 µL einer 10 mg/mL-Lösung aus Amino-HES12KD **3** in einem Puffer aus 10 mM Natriumphosphat und 150 mM Natriumchlorid, pH 7.44, wurden 1 mg SMCC **4** gelöst in 50 µL wasserfreiem DMSO gegeben und das Gemisch 80 min bei Raumtemperatur und 10 min bei 46°C behandelt. Dann wurde das Gemisch abzentifugiert, der Überstand vom Niederschlag abgenommen und erneut zentrifugiert. Vom Überstand wurden 200 µl abgenommen und 45 min bei 14000 g mit einem MICROCON YM-3 (Amicon, Millipore GmbH, Eschborn, D) Zentrifugen-Dialyseeinsatz zentrifugiert. Nach Zugabe von 400 µL Puffer aus 10 mM Natriumphosphat und 150 mM Natiurmchlorid, pH 7.44 wurde erneut für 45 min zentrifugiert, weitere 400 µL Puffer zugegeben und 60 min zentrifugiert. Die im Dialyseeinsatz verbliebene Menge an Konjugatlösung wurde auf 50 µL aufgefüllt. Von dieser Lösung wurden 10 µL zu 10 µL der Thio-DNA-Lösung. **1** gegeben und 14 h bei Raumtemperatur reagieren lassen. Zur Analyse wurden 1 µL abgenommen. Die Ergebnisse zeigt Fig. 11 in Bahn 2 und 3.

Die Reaktionsbedingungen für den beschriebenen Versuch und für Versuche mit abgewandelten Reaktionsbedingungen sind in der Tabelle 1 zusammengefasst und die Ergebnisse im Fig. 11 dargestellt.

### Zusammenfassung der Ergebnisse:

Es wurden folgende Bedingungen untersucht:
1. Menge an SMCC: 1 mg (Bahnen 2, 6, 10, 14) bzw. 5.6 mg (Bahnen 4, 8, 12, 16);
2. Temperatur für die Reaktion mit der Thio-DNA **1**:
   Raumtemperatur (Bahnen 2, 4, 6, 8) bzw. 37°C (Bahnen 10, 12, 14, 16) ;
3. Pufferbedingungen:
   10 mM Phosphat, 150 mM NaCl ohne EDTA, pH 7.44 (Bahnen 2, 4, 10, 12) bzw;
   100 mM Phosphat, 150 mM NaCl + 50 mM EDTA, pH 7,23 (Bahnen 6, 8, 14, 16)

In Figur 11, Bahnen 2-18 sind die Ergebnisse der 8 Kopplungsversuchen von Amino-HES12KD **3** an die Thio-DNA **1** mit Hilfe von SMCC dargestellt. Dabei sind die Ergebnisse direkt aus der Reaktion bzw. nach der Reaktion und anschließendem Schneiden der DNA nebeneinander dargestellt. In Bahn 1 ist ein Gemisch verschiedener Referenz-DNAs als Längenmarker aufgetragen und die Bahnen 18 und 19 zeigen Thio-DNA **1** bzw. geschnittene Thio-DNA **1**. Alle Versuche zeigen zusätzlich zu noch nicht umgesetzter Thio-DNA **1** Kopplungsprodukte bei höheren Massen (Bahnen 2, 4, 6, 8, 10, 12, 14, 16). Da HES12KD ein Gemisch unterschiedlich großer Moleküle ist, zeigen auch die Kopplungsprodukte eine Molgewichtsverteilung. Alle Kopplungsprodukte enthalten intakte DNA, da sie von EcoR1 vollständig verdaut werden können. Dies zeigt sich im fast vollständigen Verschwinden der entsprechenden diffusen Banden nach dem Schneiden (Bahnen 3, 5, 7, 9, 11, 13, 15, 17).

**Tabelle 1:Reaktionsbedingungen der Kopplung von Amino-HES12KD 3 an die Thio-DNA 1**

| **Bahn** | **Versuch** | **Temp [°C]** | **SMCC[mg]** | **DNA** | **HES12KD** | **Puffer** | |
|---|---|---|---|---|---|---|---|
| 1 | Marker | | | | | | |
| 2 | 19A1 | RT | 1 | Thio-DNA | Amino-HES12KD | 7.44, 10mM | |
| 3 | | | | | | | geschnitten |
| 4 | 19B1 | RT | 5.6 | Thio-DNA | Amino-HES12KD | 7.44, 10mM | |
| 5 | | | | | | | geschnitten |
| 6 | 19C1 | RT | 1 | Thio-DNA | Amino-HES12KD | 7.23, 100mM | |
| 7 | | | | | | | geschnitten |
| 8 | 1901 | RT | 5.6 | Thio-DNA | Amino-HES12KD | 7.23, 100mM | |
| 9 | | | | | | | geschnitten |
| 10 | 19A2 | 37°C | 1 | Thio-DNA | Amino-HES12KD | 7.44, 10mM | |
| 11 | | | | | | | geschnitten |
| 12 | 19B2 | 37°C | 5.6 | Thio-DNA | Amino-HES12KD | 7.44, 10mM | |
| 13 | | | | | | | geschnitten |
| 14 | 19C2 | 37°C | 1 | Thio-DNA | Amino-HES12KD | 7.23, 100mM | |
| 15 | | | | | | | geschnitten |
| 16 | 19D2 | 37°C | 5.6 | Thio-DNA | Amino-HES12KD | 7.23, 100mM | |
| 17 | | | | | | | geschnitten |
| 18 | | | | Thio-DNA | | | |
| 19 | | | | | | | geschnitten |

## Patentansprüche

1. Verfahren zur Herstellung eines kovalenten HAS-Wirkstoff-Konjugates, bei dem man HAS und einen Wirkstoff selektiv über die reduzierende Endgruppe in einem Reaktionsmedium miteinander umsetzt, wobei die Umsetzung über die selektiv oxidierte reduzierende Endgruppe der HAS erfolgt oder wobei HAS und Wirkstoff unmittelbar unter Entstehung einer Schiffschen Base miteinander umgesetzt werden, **dadurch gekennzeichnet, dass** das Reaktionsmedium Wasser oder ein Gemisch von Wasser mit organischem Lösungsmittel, welches mindestens 10 Gew.-% Wasser umfasst, ist, wobei der Wirkstoff ein Protein, Oligo- oder Polypeptid ist.

2. Verfahren nach Anspruch 1, bei dem man Hydroxyethylstärke mit einem mittleren Molekulargewicht (Gewichtsmittel) von 1 bis 300 kDa verwendet.

3. Verfahren nach Anspruch 1 oder 2, bei dem man Hydroxyethylstärke mit einem mittleren Molekulargewicht (Gewichtsmittel) von 1 bis 150 kDa verwendet.

4. Verfahren nach einem der Ansprüche 1 bis 3, bei dem man Hydroxyethylstärke mit einem mittleren Molekulargewicht (Gewichtsmittel) von 2 bis 40 kDa verwendet.

5. Verfahren nach einem der Ansprüche 1 bis 4, bei dem man Hydroxyethylstärke mit einem mittleren Substitutionsgrad von 0,1 bis 0,8 und einem Verhältnis von C₂:C₆-Substitution im Bereich von 2 bis 20, jeweils bezogen auf die Hydroxyethylgruppen, verwendet.

6. Verfahren nach einem der Ansprüche 1 bis 5, bei dem der Wirkstoff ein Enzym, Enzym-Inhibitor, Rezeptor, Rezeptor-Fragment, Insulin, Faktor VIII, Faktor IX, Zytokin, Interferon, Interleukin, Wachstumsfaktor, Peptid-Antibiotikum, virales Hüll-Protein, Hämoglobin, Erythropoietin, Albumin, hTPA, Antikörper, Antikörperfragment oder ein Einzelketten-Antikörper ist.

7. Verfahren nach einem der Ansprüche 1 bis 5, bei dem der Wirkstoff ein Impfstoff, ein Antibiotikum oder ein Antidiabetikum ist.

8. Verfahren nach einem der Ansprüche 1 bis 7, bei dem man eine Aminogruppe des Wirkstoffs mit HAS so umsetzt, dass eine Schiffsche Base gebildet wird.

9. Verfahren nach Anspruch 8 bei dem man HAS an eine ε-NH₂-Gruppe, α-NH₂-Gruppe oder -C(NH₂)₂-Gruppe des Wirkstoffs bindet.

10. Verfahren nach Anspruch 8 oder 9, bei dem die Azomethingruppe -CH=N- der Schiffschen Base zu einer Methylenamingruppe -CH₂-NH- reduziert wird.

11. Verfahren nach Anspruch 10, bei dem als Reduktionsmittel BH₄ verwendet wird.

12. Verfahren nach einem der Ansprüche 1 bis 7, bei dem man die reduzierende Endgruppe der HAS vor Bindung an den Wirkstoff selektiv oxidiert.

13. Verfahren nach Anspruch 12, bei dem man HAS an eine ε-NH₂-Gruppe, α-NH₂-Gruppe, SH-Gruppe, COOH-Gruppe oder -C(NH₂)₂-Gruppe des Wirkstoffs bindet.

14. Verfahren nach Anspruch 12 oder 13, bei dem die oxidierte reduzierende Endgruppe der HAS mit einer Aminogruppe des Wirkstoffs unter Ausbildung eines Amids reagiert.

15. Verfahren nach einem der Ansprüche 12 bis 14, bei dem man die HAS vor der Herstellung des Konjugates an einen Linker bindet.

16. Verfahren nach einem der Ansprüche 12 bis 14, bei dem man den Wirkstoff vor der Herstellung des Konjugates an einen Linker bindet.

17. Verfahren zur Herstellung eines Arznei- oder Diagnosemittels, umfassend Schritte, bei denen man ein kovalentes HAS-Wirkstoff Konjugat gemäß einem der Ansprüche 1 bis 16 herstellt und mit einem pharmazeutisch verträglichen Träger, Adjuvans oder Hilfsstoff vermischt.

18. HAS-Wirkstoff Konjugat, bei dem der Wirkstoff unmittelbar an die HAS kovalent gebunden ist, erhältlich durch ein Verfahren gemäß einem der Ansprüche 8 bis 11.

19. HAS-Wirkstoff Konjugat, bei dem der Wirkstoff kovalent über eine Azomethingruppe -CH=N- selektiv über die reduzierende Endgruppe der HAS unmittelbar an HAS gebunden ist, wobei der Wirkstoff ein Protein, Oligo- oder Polypeptid ist.

20. HAS-Wirkstoff Konjugat, bei dem der Wirkstoff kovalent über eine Methylenamingruppe -CH₂-NH- selektiv über die reduzierende Endgruppe der HAS unmittelbar an HAS gebunden ist, wobei der Wirkstoff ein Protein, Oligo- oder Polypeptid ist.

21. HAS-Wirkstoff-Konjugat nach Anspruch 19 oder 20, bei dem HAS an eine ε-NH₂-Gruppe, α-NH₂-Gruppe oder -C(NH₂)₂-Gruppe des Wirkstoffs gebunden ist.

22. HAS-Wirkstoff-Konjugat nach einem der Ansprüche 19 bis 21, bei dem man Hydroxyethylstärke mit einem mittleren Molekulargewicht (Gewichtsmittel) von 1 bis 150 kDa verwendet.

23. HAS-Wirkstoff-Konjugat nach einem der Ansprüche 19 bis 22, bei dem man Hydroxyethylstärke mit einem mittleren Substitutionsgrad von 0,1 bis 0,8 und einem Verhältnis von C₂:C₆-Substitution im Bereich von 2 bis 20, jeweils bezogen auf die Hydroxyethylgruppen, verwendet.

24. HAS-Wirkstoff-Konjugat nach einem der Ansprüche 19 bis 23, bei dem der Wirkstoff ein Enzym, Enzym-Inhibitor, Rezeptor, Rezeptor-Fragment, Insulin, Faktor VIII, Faktor IX, Zytokin, Interferon, Interleukin, Wachstumsfaktor, Peptid-Antibiotikum, virales Hüll-Protein, Hämoglobin, Erythropoietin, Albumin, hTPA, Antikörper, Antikörperfragment oder ein Einzelketten-Antikörper ist.

25. HAS-Wirkstoff-Konjugat nach einem der Ansprüche 19 bis 23, bei dem der Wirkstoff ein Impfstoff, ein Antibiotikum ein Antidiabetikum ist.

26. Pharmazeutische Zusammensetzung, welche eine Verbindung gemäß einem der Ansprüche 18 bis 25 aufweist.

27. Pharmazeutische Zusammensetzung nach Anspruch 26, die ferner einen pharmazeutisch verträglichen Träger aufweist.

## Claims

1. A process for the preparation of a covalent HAS-active ingredient conjugate, wherein HAS and the active ingredient are selectively coupled to each other via the reducing end group in a reaction medium, wherein HAS is coupled via its selectively oxidized reducing end group or wherein HAS and the active ingredient are directly coupled to each other thereby forming a Schiff's base, **characterized in that** the reaction medium is water or a mixture of water with an organic solvent, which comprises at least 10 weight percent of water and wherein the active ingredient is a protein, oligopeptide or polypeptide.

2. The process according to claim 1, wherein hydroxyethyl starch with an average molecular weight (weight average) of 1 to 300 kDa is used.

3. The process according to claim 1 or 2, wherein hydroxyethyl starch with an average molecular weight (weight average) of 1 to 150 kDa is used.

4. The process according to any of claims 1 to 3, wherein hydroxyethyl starch with an average molecular weight (weight average) of 2 to 40 kDa is used.

5. The process according to any of claims 1 to 4, wherein hydroxyethyl starch having an average molecular degree of substitution of 0.1 to 0.8 and a ratio of C₂ : C₆ substitution in the range of from 2 to 20, each relative to the hydroxyethyl groups, is used.

6. The process according to any of claims 1 to 5, wherein the active ingredient is an enzyme, enzyme inhibitor, receptor, receptor-fragment, insulin, factor VIII, factor IX, cytokine, interferon, interleukin, growth factor, peptide-antibiotic, viral coat protein, hemoglobin, erythropoietin, albumin, hTPA, antibody, antibody-fragment or a single chain antibody.

7. The process according to any of claims 1 to 5, wherein the active ingredient is a vaccine, an antibiotic or an antidiabetic.

8. The process according to any of claims 1 to 7, wherein an amino group of the active ingredient is reacted with HAS thereby forming a Shift base.

9. The process according to claim 8, wherein HAS is coupled to an ε-NH₂-group, α-NH₂-group or -C(NH₂)₂-group of the active ingredient.

10. The process according to claim 8 or 9, wherein the azomethine group -CH=N- of the Schiff base is reduced to a methylene amino group -CH₂-NH-.

11. The process according to claim 10, wherein BH₄- is used as reducing agent.

12. The process according to any of claims 1 to 7, wherein the reducing end group of HAS is electively oxidized before coupling to the active ingredient.

13. The process according to claim 12, wherein HAS is coupled to an ε-NH₂-group, α-NH₂-group, SH-group, COOH-group or -C(NH₂)₂-group of the active ingredient.

14. The process according to claim 12 or 13, wherein the oxidized reducing end group of HAS is coupled to an amino group of the active ingredient thereby forming an amide.

15. The process according to any of claims 12 to 14, wherein HAS is coupled to a linker prior to the preparation of the conjugate.

16. The process according to any of claims 12 to 14, wherein the active ingredient is coupled to a linker prior to the preparation of the conjugate.

17. The process for the preparation of a pharmaceutical or diagnostic agent comprising steps, wherein a covalent HAS active ingredient conjugate according to any of claims 1 to 16 is prepared and mixed with a pharmaceutically acceptable carrier, adjuvant or auxiliary compound.

18. A HAS active ingredient conjugate, wherein the active ingredient is directly covalently linked to HAS, obtainable by the method according to any of claims 8 to 11.

19. A HAS active ingredient conjugate, wherein the active ingredient is directly covalently linked to HAS via an azomethine group -CH=NH- selectively via the reducing end group of HAS, wherein the active ingredient is a protein, an oligopeptide or a polypeptide.

20. A HAS active ingredient conjugate, wherein the active ingredient is directly covalently linked to HAS via a methylene amino group -CH₂-NH- selectively via the reducing end group of HAS, wherein the active ingredient is a protein, an oligopeptide or a polypeptide.

21. The HAS active ingredient conjugate according to claim 19 or 20, wherein HAS is coupled to an ε-NH₂-group, α-NH₂-group or-C(NH₂)₂-group of the active ingredient.

22. The HAS active ingredient conjugate according to any of claims 19 to 21, wherein a hydroxyethyl starch having an average molecular weight (weight average) of 1 to 150 kDa is used.

23. The HAS active ingredient conjugate according to any of claims 19 to 22, wherein a hydroxyethyl starch having an average molecular degree of substitution of 0.1 to 0.8 and a ratio of C₂ : C₆ substitution in the range of 2 to 20, each related to the hydroxethyl groups, is used.

24. The HAS active ingredient conjugate according to any one of claims 19 to 23, wherein the active ingredient is an enzyme, enzyme inhibitor, receptor, receptor-fragment, insulin, factor VIII, factor IX, cytokine, interferon, interleukin, growth factor, peptide-antibiotic, viral coat protein, hemoglobin, erythropoietin, albumin, hTPA, antibody, antibody-fragment or a single chain antibody.

25. The HAS active ingredient conjugate according to any of claims 19 to 23, wherein the active ingredient is a vaccine, an antibiotic or an antidiabetic.

26. A pharmaceutical composition comprising a compound according to any of claims 18 to 25.

27. The pharmaceutical composition according to claim 26, additionally comprising a pharmaceutically acceptable carrier.

## Revendications

1. Procédé pour la préparation d'un conjugué covalent HAS (hydroxyalkylamidon)-substance active, dans lequel on fait réagir l'un avec l'autre HAS et une substance active sélectivement par le groupe terminal réducteur dans un milieu réactionnel, la réaction s'effectuant par le groupe terminal réducteur oxydé sélectivement de l'HAS ou HAS et la substance active réagissant l'un avec l'autre directement avec formation d'une base de Schiff, **caractérisé en ce que** le milieu réactionnel est l'eau ou un mélange d'eau et d'un solvant organique, qui comprend au moins 10 % en poids d'eau, la substance active étant une protéine, un oligo- ou polypeptide.

2. Procédé selon la revendication 1, dans lequel on utilise un hydroxyéthylamidon ayant une masse moléculaire moyenne (moyenne en poids) de 1 à 300 kDa.

3. Procédé selon la revendication 1 ou 2, dans lequel on utilise un hydroxyéthylamidon ayant une masse moléculaire moyenne (moyenne en poids) de 1 à 150 kDa.

4. Procédé selon l'une quelconque des revendications 1 à 3, dans lequel on utilise un hydroxyéthylamidon ayant une masse moléculaire moyenne (moyenne en poids) de 2 à 40 kDa.

5. Procédé selon l'une quelconque des revendications 1 à 4, dans lequel on utilise un hydroxyéthylamidon ayant un degré moyen de substitution de 0,1 à 0,8 et un rapport de substitution C₂:C₆ dans la plage de 2 à 20, chaque fois sur la base des groupes hydroxyéthyle.

6. Procédé selon l'une quelconque des revendications 1 à 5, dans lequel la substance active est une enzyme, un inhibiteur d'enzyme, un récepteur, un fragment de récepteur, l'insuline, le facteur VIII, le facteur IX, une cytokine, un interféron, une interleukine, un facteur de croissance, un antibiotique peptidique, une protéine d'enveloppe virale, l'hémoglobine, l'érythropoïétine, l'albumine, hTPA, un anticorps, un fragment d'anticorps ou un anticorps monocaténaire.

7. Procédé selon l'une quelconque des revendications 1 à 5, dans lequel la substance active est un vaccin, un antibiotique ou un antidiabétique.

8. Procédé selon l'une quelconque des revendications 1 à 7, dans lequel on fait réagir un groupe amino de la substance active avec HAS, de sorte qu'une base de Schiff est formée.

9. Procédé selon la revendication 8, dans lequel HAS se lie à un groupe ε-NH₂, un groupe α-NH₂ ou un groupe -C(NH₂)₂ de la substance active.

10. Procédé selon la revendication 8 ou 9, dans lequel le groupe azométhine -CH=N- de la base de Schiff est réduit en un groupe méthylènamino -CH₂-NH-.

11. Procédé selon la revendication 10, dans lequel on utilise comme réducteur BH₄⁻.

12. Procédé selon l'une quelconque des revendications 1 à 7, dans lequel on oxyde le groupe terminal réducteur de l'HAS avant la liaison à la substance active.

13. Procédé selon la revendication 12, dans lequel on lie HAS à un groupe ε-NH₂, un groupe α-NH₂, un groupe SH, un groupe COOH ou un groupe -C(NH₂)₂- de la substance active.

14. Procédé selon la revendication 12 ou 13, dans lequel le groupe terminal réducteur oxydé de l'HAS réagit avec un groupe amino de la substance active avec formation d'un amide.

15. Procédé selon l'une quelconque des revendications 12 à 14, dans lequel on lie l'HAS à un segment de liaison avant la préparation du conjugué.

16. Procédé selon l'une quelconque des revendications 12 à 14, dans lequel on lie la substance active à un segment de liaison avant la préparation du conjugué.

17. Procédé pour la fabrication d'un médicament ou d'un agent de diagnostic, comprenant les étapes dans lesquelles on prépare un conjugué covalent HAS-substance active selon l'une quelconque des revendications 1 à 16 et on le mélange avec un véhicule, adjuvant ou excipient pharmaceutiquement acceptable.

18. Conjugué HAS-substance active, dans lequel la substance active est directement liée par covalence à l'HAS, pouvant être obtenu par un procédé selon l'une quelconque des revendications 8 à 11.

19. Conjugué HAS-substance active, dans lequel la substance active est directement liée par covalence à HAS par un groupe azométhine -CH=N- sélectivement par le groupe terminal réducteur de l'HAS, la substance active étant une protéine, un oligo- ou polypeptide.

20. Conjugué HAS-substance active, dans lequel la substance active est directement liée par covalence à HAS par un groupe méthylènamino -CH₂-NH- sélectivement par le groupe terminal réducteur de l'HAS, la substance active étant une protéine, un oligo- ou polypeptide.

21. Conjugué HAS-substance active selon la revendication 19 ou 20, dans lequel HAS est lié à un groupe ε-NH₂, un groupe α-NH₂ ou un groupe -C(NH₂)₂ de la substance active.

22. Conjugué HAS-substance active selon l'une quelconque des revendications 19 à 21, dans lequel on utilise un hydroxyéthylamidon ayant une masse moléculaire moyenne (moyenne en poids) de 1 à 150 kDa.

23. Conjugué HAS-substance active selon l'une quelconque des revendications 19 à 22, dans lequel on utilise un hydroxyéthylamidon ayant un degré moyen de substitution de 0,1 à 0,8 et un rapport de substitution C₂:C₆ dans la plage de 2 à 20, chaque fois sur la base des groupes hydroxyéthyle.

24. Conjugué HAS-substance active selon l'une quelconque des revendications 19 à 23, dans lequel la substance active est une enzyme, un inhibiteur d'enzyme, un récepteur, un fragment de récepteur, l'insuline, le facteur VIII, le facteur IX, une cytokine, un interféron, une interleukine, un facteur de croissance, un antibiotique peptidique, une protéine d'enveloppe virale, l'hémoglobine, l'érythropoiétine, l'albumine, hTPA, un anticorps, un fragment d'anticorps ou un anticorps monocaténaire.

25. Conjugué HAS-substance active selon l'une quelconque des revendications 19 à 23, dans lequel la substance active est un vaccin, un antibiotique ou un antidiabétique.

26. Composition pharmaceutique, qui comporte un composé selon l'une quelconque des revendications 18 à 25.

27. Composition pharmaceutique selon la revendication 26, qui comporte en outre un véhicule pharmaceutiquement acceptable.
